# EUROPEAN PATENT APPLICATION

(11) **EP 4 403 169 A1**
(43) Date of publication of application: **24.07.2024**
(21) Application number: 22869966.6
(22) Date of filing: 13.09.2022
(51) Int. Cl.: A61K 31/015, A24D 3/04, A24D 3/14, A61K 9/08, A61K 9/48, A61K 9/72, A61K 47/10, A61K 47/14, A61K 47/44, C07C 13/32, C11B 9/00

(54) **CARYOPHYLLENE-CONTAINING COMPOSITION**

(30) Priority: 14.09.2021 JP 2021149802
(71) Applicant: Sunsho Pharmaceutical Co., Ltd., Fuji-shi, Shizuoka 419-0201 (JP)
(72) Inventor: ZAIMA, Nobuhiro, Nara-shi, Nara 631-8505 (JP); MATSUMURA, Shinichi, Osaka-shi, Osaka 532-0027 (JP); IWAMOTO, Kohei, Osaka-shi, Osaka 532-0027 (JP); YOSHIOKA, Yuri, Osaka-shi, Osaka 532-0027 (JP); YAMADA, Kazuya, Fuji-shi, Shizuoka 419-0201 (JP); TSUNODA, Yamato, Fuji-shi, Shizuoka 419-0201 (JP); TOYOTA, Kohei, Fuji-shi, Shizuoka 419-0201 (JP); ITO, Masakazu, Fuji-shi, Shizuoka 419-0201 (JP); SUZUKI, Tomoya, Fuji-shi, Shizuoka 419-0201 (JP)
(74) Representative: Kraus & Lederer PartGmbB
(86) International application number: PCT/JP2022/034212
(87) International publication number: WO 2023/042819

(57) **Abstract**

Provided are a novel composition and others. The composition at least comprises caryophyllene, and menthol and/or a fat or oil.

## Description

### TECHNICAL FIELD

The present invention relates to a caryophyllene-containing composition and others.

### BACKGROUND ART

Caryophyllene (β-caryophyllene) is known to be capable of, for example, reducing anxiety (see Patent Literature 1).

### CITATION LIST

### Patent Literature

Patent Literature 1: JP-A 2006-342062

### SUMMARY OF INVENTION

### TECHNICAL PROBLEM

As described above, caryophyllene is known to be capable of reducing anxiety, but the effects of caryophyllene on other substances such as menthol and fats or oils [such as medium-chain triglycerides (MCTs)] remain unknown.

In response to this issue, an object of the present invention is to provide a novel composition comprising caryophyllene, novel functions of caryophyllene, and others.

### SOLUTION TO PROBLEM

After extensive research to achieve the above-mentioned object, the present inventors found that caryophyllene can enhance volatilization and dissolution of menthol and can improve freezing resistance in the form of a composition constituted together with menthol and a fat or oil (can form a composition having excellent freezing resistance together with menthol and a fat or oil). The present inventors conducted further investigation and then completed the present invention.

That is, the present invention relates to the following.
[1] A composition comprising caryophyllene and menthol.
[2] A composition comprising caryophyllene and a fat or oil.
[3] A composition comprising caryophyllene, menthol, and a fat or oil.
[4] The composition according to the above [2] or [3], wherein the fat or oil comprises a medium-chain triglyceride (MCT).
[5] The composition according to any one of the above [1] to [4] , wherein the amount of the caryophyllene is 1 mass% or more.
[6] The composition according to any one of the above [1] and [3] to [5], wherein the amount of the caryophyllene is 3 mass% or more, and wherein the amount of the caryophyllene relative to the combined amount of the caryophyllene and the menthol is 5 to 95 mass%.
[7] The composition according to any one of the above [2] to [6] , wherein the amount of the caryophyllene is 3 mass% or more, and wherein the amount of the caryophyllene relative to the combined amount of the caryophyllene and the fat or oil is 5 to 95 mass%.
[8] The composition according to any one of the above [3] to [7] , wherein the amount of the caryophyllene is 3 mass% or more, and wherein the amount of the caryophyllene relative to the combined amount of the caryophyllene, the menthol, and the fat or oil is 5 to 95 mass%.
[9] The composition according to any one of the above [1] and [3] to [8], wherein the amount of the menthol is 15 mass% or more.
[10] The composition according to any one of the above [1] to [9], further comprising at least one ingredient (X) selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms.
[11] The composition according to any one of the above [1] to [10], further comprising a dicarboxylic acid ester as the ingredient (X).
[12] The composition according to the above [10] or [11], wherein the amount of the ingredient (X) is 1 mass% or more.
[13] The composition according to any one of the above [10] to [12], wherein the amount of the ingredient (X) is 3 mass% or more, and wherein the amount of the ingredient (X) relative to the combined amount of the caryophyllene and the ingredient (X) is 5 to 95 mass%.
[14] The composition according to any one of the above [1] to [13], wherein the composition is in a liquid form.
[15] The composition according to any one of the above [1] to [14] , wherein the composition is intended for use for a capsule content (core) (wherein the composition is used for a capsule content or forms a capsule content).
[16] A capsule having a core and a shell, wherein the core is formed of the composition according to any one of the above [1] to [15].
[17] A filter at least containing a capsule, wherein the capsule has a core and a shell, and wherein the core is formed of the composition according to any one of the above [1] to [15].
[18] A tobacco product containing the composition according to any one of the above [1] to [15].
[19] An inhalation device containing the composition according to any one of the above [1] to [15].
[20] The inhalation device according to the above [19], wherein the inhalation device is a smoking device.
[21] The tobacco product or inhalation device according to any one of the above [18] to [20], wherein the tobacco product or inhalation device contains the capsule according to the above [16] or the filter according to the above [17].
[22] An agent for enhancing (improving) volatilization (volatilization rate) of menthol (or menthol in a menthol-containing composition), comprising caryophyllene.
[23] An agent for enhancing (improving) dissolution (dissolution rate) of menthol (or menthol in a menthol-containing composition), comprising caryophyllene.
[24] An agent for enhancing (improving) freezing resistance of a fat or oil, comprising caryophyllene.
[25] A method for enhancing (improving) volatilization and/or dissolution of menthol, comprising mixing (contacting) caryophyllene (a caryophyllene-containing agent) with the menthol (a menthol-containing composition).
[26] A method for enhancing freezing resistance of a fat or oil, comprising mixing (contacting or combining) caryophyllene (a caryophyllene-containing agent) with the fat or oil (a fat-or-oil-containing composition).
[27] A method for pulmonary delivery of caryophyllene (and menthol), comprising using the capsule, the filter, the tobacco product, and/or the inhalation device according to any one of the above [16] to [21].

### ADVANTAGEOUS EFFECTS OF INVENTION

The present invention provides a novel composition comprising caryophyllene, novel functions of caryophyllene, and others.

For example, one aspect of the invention provides a composition comprising caryophyllene and menthol, an agent comprising caryophyllene for enhancing (improving, facilitating) volatilization or dissolution of menthol (or a menthol-containing composition), an agent comprising caryophyllene for enhancing (improving, facilitating) freezing resistance of menthol (or a menthol-containing composition), and others.

Caryophyllene, when used in combination with menthol, can enhance volatilization or dissolution of the menthol (or enables better volatilization or dissolution of the menthol in the form of a composition constituted with the menthol) and can enhance freezing resistance of the menthol (or enables better freezing resistance of the menthol in the form of a composition constituted with the menthol) . Therefore, the composition and the agents described above can be provided.

Another aspect of the invention provides a composition comprising caryophyllene and a fat or oil, an agent comprising caryophyllene for enhancing (improving, facilitating) freezing resistance of a fat or oil (or a fat-or-oil-containing composition), and others.

Caryophyllene, when used in combination with a fat or oil (such as an MCT), can enhance freezing resistance of the fat or oil (or enables better freezing resistance of the fat or oil in a composition constituted with the fat or oil) . Therefore, the composition and the agent described above can be provided.

### DESCRIPTION OF EMBODIMENTS

### Caryophyllene

The composition of the present invention comprises caryophyllene. Similarly, the agent of the present invention and specific use (applications) according to the present invention such as a capsule, a filter, and an inhalation device comprise caryophyllene.

Examples of the caryophyllene include β-caryophyllene, α-caryophyllene, isocaryophyllene, and metabolites or derivatives of caryophyllene (e.g., caryophyllene oxides such as β-caryophyllene oxide). The caryophyllene may comprise a single type of caryophyllene or a combination of two or more types of caryophyllenes selected from the above examples.

The caryophyllene usually may at least comprise β-caryophyllene. The caryophyllene may comprise β-caryophyllene and a type of caryophyllene that is not β-caryophyllene [e.g., at least one selected from α-caryophyllene, isocaryophyllene, and metabolite or derivatives of caryophyllene] .

The amount of β-caryophyllene in the caryophyllene at least comprising β-caryophyllene is, for example, 30 mass% or more, 50 mass% or more, 70 mass% or more, 80 mass% or more, 90 mass% or more, 95 mass% or more, 100 mass% (substantially 100 mass%), etc.

As used herein, the term "β-caryophyllene" is sometimes used to collectively refer to all caryophyllenes, including those that are not β-caryophyllene.

The caryophyllene (β-caryophyllene) is not particularly limited and may be derived (e.g., extracted or concentrated) from, for example, clove, caraway, basil, oregano, hop, cinnamon, Ceylon cinnamon, rosemary, cannabis, hemp, *cannabis sativa,* black pepper, lavender, malabathrum, ylang-ylang, copaiba, Guinea ginger, curry leaf, or other essential oils.

The caryophyllene may be a commercial product or may be produced (purified) by commonly used methods (chemically synthesized).

The amount of the caryophyllene is not particularly limited and can be selected according to the desired function (e.g., enhanced volatilization, enhanced dissolution, freezing resistance, as well as other functions of caryophyllene), the dosage form of the composition, and other factors. For example, the amount (proportion or concentration) of the caryophyllene in the composition may be 0.01 mass% (% by weight, wt%, the same hereinafter) or more (e.g., 0.05 mass% or more), 0.1 mass% or more (e.g., 0.5 mass% or more), 1 mass% or more (e.g., 5 mass% or more), 10 mass% or more (e.g., 15 mass% or more), 20 mass% or more (e.g., 25 mass% or more), 30 mass% or more (e.g., 35 mass% or more), 40 mass% or more (e.g., 45 mass% or more), 50 mass% or more (e.g., 55 mass% or more), 60 mass% or more (e.g., 65 mass% or more), 70 mass% or more (e.g., 75 mass% or more), 80 mass% or more (e.g., 85 mass% or more), 90 mass% or more (e.g., 95 mass% or more), etc. In addition, the amount of the caryophyllene in the composition may be 99.9 mass% or less (e.g., 99.5 mass% or less), 99 mass % or less (e.g., 95 mass % or less), 90 mass% or less, 80 mass% or less, 70 mass% or less, 60 mass% or less, 50 mass% or less, 40 mass% or less, 30 mass% or less, etc.

These lower and upper limits may be combined to set an appropriate range (e.g., 0.1 to 90 mass%, 10 to 50 mass%, etc.) for the concentration (amount) of the caryophyllene [the same applies to defining the range described herein (e.g., the ranges of menthol, a fat or oil, and others, which are described later)]. More specifically, the concentration (concentration range) of the caryophyllene is, for example, 1 mass% or more, 3 to 99 mass%, 5 to 80 mass%, 5 to 90 mass%, 15 to 30 mass%, etc.

In the case where the composition comprises menthol (or where the caryophyllene is combined with menthol), the amount (concentration) of the caryophyllene relative to the combined amount of the caryophyllene and the menthol [when the combined amount is assumed as 100 mass%] may be selected from the above-described ranges (e.g., 1 mass% or more, 10 mass% or more, 5 to 90 mass%, etc.).

Similarly, in the case where the composition comprises a fat or oil (or where the caryophyllene is combined with a fat or oil), the amount (concentration) of the caryophyllene relative to the combined amount of the caryophyllene and the fat or oil [when the combined amount is assumed as 100 mass%] may be selected from the above-described concentration ranges of the caryophyllene in the composition (e.g., 1 mass% or more, 10 mass% or more, 5 to 90 mass%, etc.).

Similarly, in the case where the composition comprises menthol and a fat or oil (or where the caryophyllene is combined with menthol and a fat or oil), the amount (concentration) of the caryophyllene relative to the combined amount of the caryophyllene, the menthol, and the fat or oil [when the combined amount is assumed as 100 mass%] may be selected from the above-described concentration ranges of the caryophyllene in the composition (e.g., 1 mass% or more, 10 mass% or more, 5 to 90 mass%, etc.) again.

In the case where the composition comprises an ingredient (X), which is described later, the amount (concentration) of the caryophyllene relative to the combined amount of the caryophyllene and the ingredient (X) [when the combined amount is assumed as 100 mass%] can be selected from the above-described concentration ranges of the caryophyllene in the composition (e.g., 5 mass% or more, 95 mass% or less, 10 to 90 mass%, etc.).

Caryophyllene is known (reported) to reduce (ameliorate or suppress) anxiety [e.g., motion sickness, nocturia, stress urticaria], reduce (ameliorate or suppress) stress, inhibit β-secretase (β-secretase activity), and prevent or improve major neurocognitive disorder (or dementia, for example, senile dementia such as Alzheimer's disease). For this reason, the composition of the present invention can be used to provide (or achieve) such functions (effects), which are appropriate according to the mode of its use.

### Menthol

The composition may comprise menthol. Menthol usually may at least comprise l-menthol or may substantially be l-menthol, depending on the desired function and other factors.

The menthol in the composition (e.g., the composition at ordinary temperature or room temperature, or the composition below the melting point of the menthol) may be dissolved (in a solid solution state) without being solidified (crystallized, purely solidified).

The amount (concentration) of the menthol in the composition can be selected according to the desired function (e.g., the function of the menthol as well as the function of the caryophyllene constituting the composition), the dosage form of the composition, and other factors, and is not particularly limited. The amount (concentration) of the menthol in the composition can usually be selected from a range of about 0.5 mass% or more (e.g., 1 mass% or more) and may be, for example, 2 mass% or more (e.g., 3 mass% or more), 5 mass% or more (e.g., 8 mass% or more), 10 mass% or more (e.g., 12 mass% or more), 15 mass% or more (e.g., 18 mass% or more), 20 mass (e.g., 22 mass% or more), 25 mass% or more (e.g., 28 mass% or more), 30 mass% or more (e.g., 32 mass% or more), 35 mass% or more (e.g., 38 mass% or more), 40 mass% or more (e.g., 42 mass% or more), 45 mass% or more (e.g., 48 mass% or more), 50 mass% or more, etc. In addition, the amount (concentration) of the menthol in the composition may be 95 mass% or less (e.g., 90 mass% or less), 80 mass% or less (e.g., 75 mass% or less), 70 mass% or less (e.g., 65 mass% or less), 60 mass% or less (e.g., 55 mass% or less), 50 mass% or less (e.g., 48 mass% or less, or 45 mass% or less), 40 mass% or less (e.g., 35 mass% or less), 30 mass% or less (e.g., 28 mass% or less), 25 mass% or less (e.g., 22 mass% or less), 20 mass% or less (e.g., 18 mass % or less), etc.

These lower and upper limits can be combined to set an appropriate range for the concentration (amount) of the menthol similarly as described above. More specifically, the range is, for example, 3 mass% or more, 5 to 70 mass%, 10 to 65 mass%, 10 to 60 mass%, 15 mass% or more, 15 to 55 mass%, 15 to 45 mass%, etc.

The concentration (amount) of the menthol may be relatively high (e.g., 10 mass% or more, 15 mass% or more, 35 mass% or more, 40 mass% or more, etc.). In the present invention, a combined use of caryophyllene with menthol enables the achievement of enhanced volatilization and dissolution of the menthol and excellent freezing resistance of the menthol, even at such a high concentration of the menthol.

The amount (concentration) of the menthol relative to the combined amount of the caryophyllene and the menthol [when the combined amount is assumed as 100 mass%] can be selected from the above-described concentration ranges of the menthol in the composition (e.g., 5 mass% or more, 80 mass% or less, 3 to 60 mass%, etc.).

Similarly, in the case where the composition comprises caryophyllene, menthol, and a fat or oil, the amount (concentration) of the menthol relative to the combined amount of the caryophyllene, the menthol, and the fat or oil [when the combined amount is assumed as 100 mass%] may be selected from the above-described concentration ranges of the menthol in the composition (e.g., 5 mass% or more, 80 mass% or more, 3 to 60 mass%, etc.).

Similarly, in the case where the composition comprises menthol and an ingredient (X), which is described later, the amount (concentration) of the menthol relative to the combined amount of the menthol and the ingredient (X) [when the combined amount is assumed as 100 mass%] can be selected from the above-described concentration ranges of the menthol in the composition (e.g., 5 mass% or more, 80 mass% or more, 3 to 60 mass%, etc.) again.

Menthol is known (reported) to function, for example, as a flavoring agent, a coolant, or an inhibitor of gastric peristalsis. For this reason, the composition of the present invention can be used to provide (or achieve) such functions (effects), which are appropriate according to the mode of its use.

### Fat or oil

The composition may comprise a fat or oil.

Examples of the fat or oil include vegetable oils (e.g., soybean oil, rapeseed oil, corn oil, sesame oil, linseed oil, cotton seed oil, perilla oil, olive oil, rice oil, palm oil, jojoba oil, sunflower oil, camellia oil, etc.), animal oils (e.g., beef fat, pork fat, chicken fat, milk fat, fish oil, horse oil, etc.), and medium-chain triglycerides (MCTs).

A single kind of fat or oil or a combination of two or more kinds of fats or oils may be used.

Among these fats or oils, MCTs are particularly preferred. For this reason, the fat or oil may at least comprise an MCT. In the case where the fat or oil comprises an MCT, the amount of the MCT relative to the total mass of the fat or oil may be, for example, 10 mass% or more, 30 mass% or more, 50 mass% or more, 70 mass% or more, 80 mass% or more, 90 mass% or more, 100 mass% (MCT only), etc.

Fats or oils can function as a medium (solvent, carrier) in the composition. In addition, some types of fats or oils (e.g., MCTs) are relatively compatible with menthol because they minimally affect (insignificantly reduce) volatilization and dissolution as well as freezing resistance of the menthol, and furthermore, such fats or oils themselves have a relatively good freezing resistance. For this reason, a portion of the amount of the caryophyllene and others can be replaced with a certain fat or oil (particularly such as MCTs) in a preferable embodiment. In addition, the use of fats or oils (such as MCTs) can be advantageous in terms of encapsulation and others. Furthermore, fats or oils hardly affect flavors and are easy to use in combination with menthol and caryophyllene.

The proportion (amount, concentration) of the fat or oil in the composition may be, for example, 0.1 mass% or more (e.g., 0.5 mass% or more), 1 mass% or more (e.g., 5 mass% or more), 10 mass% or more (e.g., 15 mass% or more), 20 mass% or more (e.g., 25 mass% or more), 30 mass% or more (e.g., 35 mass% or more), 40 mass% or more (e.g., 45 mass% or more), 50 mass% or more (e.g., 55 mass% or more), 60 mass% or more (e.g., 65 mass% or more), 70 mass% or more (e.g., 75 mass% or more), 80 mass% or more (e.g., 85 mass% or more), 90 mass% or more (e.g., 95 mass% or more), etc. In addition, the amount of the fat or oil in the composition may be 99 mass% or less (e.g., 95 mass% or less), 90 mass% or less, 80 mass% or less, 70 mass% or less, 60 mass% or less, 50 mass% or less, 40 mass% or less, 30 mass% or less, 20 mass% or less, etc.

Similarly, the amount (concentration) of the fat or oil relative to the combined amount of the caryophyllene and the fat or oil [when the combined amount is assumed as 100 mass%] may be selected from the above-described concentration ranges of the fat or oil in the composition (e.g., 1 mass% or more, 10 mass% or more, 5 to 90 mass%, etc.).

Similarly, in the case where the composition comprises menthol and a fat or oil (or where the caryophyllene is combined with menthol and a fat or oil), the amount (concentration) of the fat or oil relative to the combined amount of the caryophyllene, the menthol, and the fat or oil [when the combined amount is assumed as 100 mass%] may be selected from the above-described concentration ranges of the fat or oil in the composition (e.g., 1 mass% or more, 10 mass% or more, 5 to 90 mass%, etc.) again.

In the case where the composition comprises a fat or oil and an ingredient (X), which is described later, the amount (concentration) of the fat or oil relative to the combined amount of the fat or oil and the ingredient (X) [when the combined amount is assumed as 100 mass%] can be selected from the above-described concentration ranges of the fat or oil in the composition (e.g., 5 mass% or more, 95 mass% or less, 10 to 90 mass%, etc.).

### Additional ingredients

The composition may comprise an additional ingredient (an ingredient other than the caryophyllene, the menthol, or the oil or fat).

The additional ingredient is not particularly limited and can be selected according to the form and application of the composition, the intended subject of the composition, and other factors. Examples include carriers, excipients, binders, disintegrants, lubricants, coating agents, colorants, flavoring agents, stabilizers, emulsifiers (surfactants), absorption enhancers, gelling agents, pH adjusters, preservatives, antioxidants, coolants, physiologically active substances, biologically active substances, microorganisms, foods and drinks, plants, sweeteners, acidulants, seasonings, and revitalizers.

A single kind of additional ingredient or a combination of two or more kinds of additional ingredients may be used.

Examples of the carrier (medium, non-fat or non-oil carrier) include acids (e.g., fatty acids such as caprylic acid, capric acid, eicosapentaenoic acid, docosahexaenoic acid, oleic acid, and linoleic acid), hydrocarbons (e.g., liquid paraffin, squalane, and vaseline), silicones (e.g., silicone oil, etc.), synthetic polymers (e.g., polyacrylic acid, carboxyvinyl polymer, polyethylene glycol, polyvinylpyrrolidone, etc.), natural polymers or their derivatives (e.g., carrageenan, alginic acid, cellulose, guar gum, xanthan gum, quince seed, dextran, gellan gum, hyaluronic acid, ethyl cellulose, hydroxypropyl cellulose, hydroxypropyl methyl cellulose, cationic guar gum, acetylated hyaluronic acid, sodium alginate, etc.), lower alcohols (e.g., ethanol, isopropanol, etc.), polyhydric alcohols (e.g., ethylene glycol, glycerol, propylene glycol, butylene glycol, diglycerol, and dipropylene glycol), and water.

The properties of the carrier can be selected according to the form of the composition and other factors. The carrier may be in a solid, liquid, or some other form, and may be non-volatile or volatile. A liquid carrier can be a solvent.

The emulsifier (surfactant) is not particularly limited, and examples include nonionic surfactants [e.g., sugar fatty acid esters (e.g., sucrose fatty acid esters, maltose fatty acid esters, and lactose fatty acid esters), propylene glycol fatty acid esters, glycerol fatty acid esters, sorbitan fatty acid esters, polyglycerol fatty acid esters, and organic acid monoglycerides].

In the case where the emulsifier is used, the amount (concentration) of the emulsifier in the composition can be selected according to the mode and application of the composition, and other factors, and is not particularly limited. For example, the amount (concentration) of the emulsifier in the composition can be selected from a range of about 40 mass% or less (e.g., 35 mass% or less) and is, for example, 30 mass% or less (e.g., 25 mass% or less), preferably 20 mass% or less (e.g., 15 mass% or less), and more preferably 10 mass% or less (e.g., 8 mass% or less) or 5 mass% or less (e.g., 4 mass% or less, 3 mass% or less, 1 mass% or less, etc.).

The lower limit of the amount (concentration) of the emulsifier in the composition can be selected according to the mode and application of the composition, and other factors, and is not particularly limited. The lower limit may be, for example, 0.01 mass%, 0.1 mass%, 0.5 mass%, 0.7 mass%, 1 mass%, 1.2 mass%, 1.5 mass%, 2 mass%, 3 mass%, etc.

The emulsifier can cause a significantly reduced interfacial tension in the composition. For this reason, when the composition comprising an emulsifier is used as a content of a capsule (e.g., a seamless capsule produced by a drop method or some other method), encapsulation is prone to failure. From this perspective, it is desirable to use no emulsifiers (substantially no emulsifiers) when the composition is used as a capsule content. If the emulsifier is used, it is desirable that its amount is relatively small (e.g., 5 mass% or less in the composition, 3 mass% or less in the composition, etc.).

The composition may comprise at least one ingredient (hereinafter referred to as "ingredient (X)") selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms.

The ingredient (X) can function as a medium (solvent, carrier). In addition, the ingredient (X) is relatively compatible with menthol because the ingredient (X) hinders neither dissolution of the menthol nor freezing resistance of the menthol or the fat or oil. In particular, a combined use of the ingredient (X) with caryophyllene can enhance dissolution of the menthol as well as freezing resistance of the menthol and the fat or oil.

For this reason, a portion of the amount of the caryophyllene can be replaced with the ingredient (X) (caryophyllene and the ingredient (X) can be used in combination). Even when the composition comprises the ingredient (X), encapsulation can often be achieved without particular trouble.

The ingredient (X) may be in a liquid or solid form at ordinary temperature or room temperature (e.g., 15 to 35°C).

A solid ingredient (X) in the composition at ordinary temperature or room temperature is often in a liquid form (e.g., dissolved in a liquid ingredient (X) or in an ingredient other than the ingredient (X)).

In particular, the ingredient (X) may at least comprise a liquid ingredient at ordinary temperature or room temperature. Typically, the ingredient (X) (or all the ingredients (X) when two kinds or more of ingredients (X) are used) is in a liquid form at ordinary temperature or room temperature.

The ingredient (X) may have the same function as that of the other types of additional ingredients described above (such as a carrier).

The ingredient (X) will be described in detail below.

### Dicarboxylic acid ester

The dicarboxylic acid ester is an ester of a dicarboxylic acid. The dicarboxylic acid ester may be a monoester (half ester) or a diester and is particularly a dicarboxylic acid diester.

The dicarboxylic acid diester is usually an ester of one dicarboxylic acid molecule and two alcohol molecules. The two alcohol molecules may be the same or different molecules. The two alcohol molecules are usually the same molecules.

The dicarboxylic acid is not particularly limited, and examples include aliphatic dicarboxylic acids and aromatic dicarboxylic acids. The aliphatic dicarboxylic acid can be a saturated or unsaturated dicarboxylic acid. The aliphatic dicarboxylic acid may be chained (including branched-chained) or cyclic. The dicarboxylic acid may be, for example, an oxycarboxylic acid.

Specific examples of the dicarboxylic acid include aliphatic dicarboxylic acids [e.g., saturated dicarboxylic acids (e.g., C₂₋₂₀ saturated dicarboxylic acids, preferably C₂₋₁₆ saturated dicarboxylic acids, more preferably C₄₋₁₂ saturated dicarboxylic acids, such as oxalic acid, malonic acid, succinic acid, glutaric acid, adipic acid, pimelic acid, suberic acid, azelaic acid, sebacic acid, and cyclohexane dicarboxylic acid), unsaturated dicarboxylic acids (e.g., C₄₋₂₀ unsaturated dicarboxylic acids, preferably C₂₋₁₆ unsaturated dicarboxylic acids, more preferably C₄₋₁₂ unsaturated dicarboxylic acids, such as fumaric acid, maleic acid, and cyclohexene dicarboxylic acid), etc.] and aromatic dicarboxylic acids [e.g., C₈₋₂₀ aromatic dicarboxylic acids, preferably C₈₋₁₆ aromatic dicarboxylic acids, preferably C₈₋₁₂ aromatic dicarboxylic acids, such as phthalic acid, isophthalic acid, and terephthalic acid] .

The alcohol is not particularly limited, and examples include aliphatic alcohols (including aromatic aliphatic alcohols) and aromatic alcohols. The alcohol may be saturated or unsaturated. The alcohol may be chained (including branched-chained) or cyclic. The alcohol may be a monool or a polyol and is usually a monool.

Specific examples of the alcohol (monool) include aliphatic alcohols [e.g., alkanols (e.g., C₁₋₂₀ alkanols, preferably C₁₋₁₂ alkanols, more preferably C₁₋₆ alkanols (e.g., C₁₋₄ alkanols), such as methanol, ethanol, propanol, isopropanol, butanol, isobutanol, tert-butanol, pentanol, hexanol, heptanol, octanol, 2-ethyl hexanol, decanol, dodecanol, and cyclohexanol), aralkyl alcohols (e.g., hydroxy C₁₋₄ alkyl C₆₋₁₀ arenes such as benzyl alcohol and phenethyl alcohol), etc.].

Examples of the dicarboxylic acid ester include esters of all combinations of these dicarboxylic acids and alcohols. Specific examples of the dicarboxylic acid diester include aliphatic dicarboxylic acid diesters {e.g., saturated dicarboxylic acid diesters [e.g., diesters (e.g., diesters with aliphatic alcohols, such as dialkyl esters (e.g., C₁₋₁₀ alkyl esters, C₁₋₆ alkyl esters, etc.)) of C₂₋₂₀ saturated dicarboxylic acids, such as diethyl malonate, dimethyl succinate, diethyl succinate, diethyl glutarate, diisobutyl adipate, dipropyl adipate, diethyl pimelate, diethyl suberate, diethyl azelate, and diethyl sebacate], unsaturated dicarboxylic acid diesters [e.g., diesters (e.g., diesters with aliphatic alcohols) of C₄₋₂₀ unsaturated dicarboxylic acids, such as diethyl fumarate], etc.} and aromatic dicarboxylic acid esters [e.g., esters (e.g., diesters with aliphatic alcohols, such as dialkyl esters) of C₈₋₂₀ aromatic dicarboxylic acids, such as diethyl phthalate] .

Among these dicarboxylic acid esters, aliphatic dicarboxylic acid diesters are particularly desirable, and from the perspective of odor and others, diesters of saturated or unsaturated dicarboxylic acids having four or more carbon atoms (e.g., diesters of C₄₋₂₀ saturated or unsaturated dicarboxylic acids, such as diethyl succinate, diethyl sebacate, diisobutyl adipate, and diethyl fumarate) are desirable.

A single kind of dicarboxylic acid diester or a combination of two or more kinds of dicarboxylic acid diesters may be used.

### Diol ester

The diol ester is an ester of a diol. The diol ester may be a monoester or diester and is particularly a diol diester.

The diol diester is usually an ester of one diol molecule and two carboxylic acid molecules. The two carboxylic acid molecules may be the same or different molecules. The two carboxylic acid molecules are usually the same molecules.

The diol is not particularly limited, and examples include aliphatic diols (including aromatic aliphatic diols) and aromatic diols. The aliphatic diol may be saturated or unsaturated. The aliphatic diol may be chained (including branched-chained) or cyclic.

Specific examples of the diol include aliphatic diols [e.g., alkanediols (e.g., C₂₋₂₀ alkanediols, preferably C₂₋₁₆ alkanediols, more preferably C₂₋₁₂ alcohols, such as ethylene glycol, 1,3-propanediol, propylene glycol, 1,4-butanediol, butylene glycol, pentanediol, hexanediol, heptanediol, octanediol, decanediol, dodecanediol, cyclohexanediol, and cyclohexanedimethanol), polyalkanediols (e.g., di- to hexa-C₂₋₆ alkanediols such as diethylene glycol, dipropylene glycol, and triethylene glycol), hydroxyalkylarenes (e.g., di(hydroxy C₁₋₄ alkyl)C₆₋₁₀ arenes such as xylylene glycol), etc.].

The carboxylic acid is not particularly limited, and examples include aliphatic carboxylic acids (including aromatic aliphatic carboxylic acids) and aromatic carboxylic acids. The carboxylic acid may be saturated or unsaturated. The carboxylic acid may be chained (including branched-chained) or cyclic. The carboxylic acid may be, for example, an oxycarboxylic acid.

The carboxylic acid may be a monocarboxylic acid or a polycarboxylic acid and is typically a monocarboxylic acid.

Specific examples of the carboxylic acid include monocarboxylic acids including aliphatic carboxylic acids (e.g., C₁₋₃₀ aliphatic carboxylic acids, preferably C₁₋₁₂ aliphatic carboxylic acids, more preferably C₁₋₆ aliphatic carboxylic acids, such as formic acid, acetic acid, propionic acid, butyric acid, valeric acid, isovaleric acid, caproic acid, enanthic acid, caprylic acid, pelargonic acid, capric acid, lauric acid, myristic acid, palmitic acid, margaric acid, stearic acid, oleic acid, and cyclohexane carboxylic acid) and aromatic carboxylic acids (e.g., carboxy C₆₋₁₀ arenes such as benzoic acid and salicylic acid).

Examples of the diol ester include esters of all combinations of these diols and carboxylic acids.

Specific examples of the diol ester include diol diesters including aliphatic diol diesters [e.g., diesters (e.g., diesters with aliphatic carboxylic acids) of C₂₋₂₀ aliphatic diols, such as ethylene glycol diacetate, propylene glycol diacetate, 1,4-butanediol diacetate, butylene glycol diacetate, 1,6-hexanediol diacetate, 1,8-octanediol diacetate, ethylene glycol bis(butyrate), and triethylene glycol dibutylate] .

Particularly preferred are diesters (e.g., diesters with aliphatic carboxylic acids) of aliphatic diols having three or more carbon atoms (e.g., C₃₋₂₀ aliphatic diols, C₄₋₁₆ aliphatic diols, C₆₋₁₂ aliphatic diols, etc.) from the perspective of the dissolution of the menthol and others.

A single kind of diol ester or a combination of two or more kinds of diol esters may be used.

### Monocarboxylic acid ester

The monocarboxylic acid ester (monocarboxylic acid monoester) is an ester of a monocarboxylic acid (an ester of a monocarboxylic acid and an alcohol, that is, an ester of one alcohol molecule and one carboxylic acid molecule). The alcohol may be a monool or a polyol and is usually a monool.

The monocarboxylic acid is not particularly limited, and examples include aliphatic carboxylic acids (including aromatic aliphatic carboxylic acids) and aromatic carboxylic acids. The carboxylic acid may be saturated or unsaturated. The carboxylic acid may be chained (including branched-chained) or cyclic. The carboxylic acid may be, for example, an oxycarboxylic acid.

Specific examples of the monocarboxylic acid include aliphatic carboxylic acids [e.g., C₁₋₃₀ aliphatic carboxylic acids, preferably C₄₋₂₈ aliphatic carboxylic acids, more preferably C₆₋₂₄ aliphatic carboxylic acids (e.g., aliphatic carboxylic acids having 14 carbon atoms or less), such as formic acid, acetic acid, propionic acid, butyric acid, valeric acid, isovaleric acid, caproic acid, enanthic acid, caprylic acid, pelargonic acid, capric acid, lauric acid, myristic acid, palmitic acid, margaric acid, stearic acid, oleic acid, linoleic acid, linolenic acid, and cyclohexane carboxylic acid] and aromatic carboxylic acids (e.g., carboxy C₆₋₁₀ arenes such as benzoic acid and salicylic acid).

The alcohol is not particularly limited, and examples include aliphatic alcohols (including aromatic aliphatic alcohols) and aromatic alcohols. The alcohol may be saturated or unsaturated. The alcohol may be chained (including branched-chained) or cyclic.

Specific examples of the alcohol (monool) include aliphatic alcohols [e.g., alkanols (e.g., C₁₋₂₀ alkanols, preferably C₁₋₁₂ alkanols, more preferably C₁₋₆ alkanols (e.g., C₁₋₄ alkanols), such as methanol, ethanol, propanol, isopropanol, butanol, isobutanol, tert-butanol, pentanol, hexanol, heptanol, octanol, 2-ethyl hexanol, decanol, dodecanol, and cyclohexanol), aralkyl alcohols (e.g., hydroxy C₁₋₄ alkyl C₆₋₁₀ arenes such as benzyl alcohol and phenethyl alcohol), etc.].

Examples of the monocarboxylic acid ester include esters of all combinations of these monocarboxylic acids and alcohols.

Specific examples of the monocarboxylic acid ester include aliphatic carboxylic acid esters [aliphatic monocarboxylic acid esters (monoesters), for example, esters (e.g., esters with aliphatic alcohols) of C₁₋₃₀ aliphatic carboxylic acids (e.g., C₄₋₂₈ aliphatic carboxylic acids, C₆₋₂₄ aliphatic carboxylic acids, etc.), such as ethyl decanoate, ethyl laurate, ethyl palmitate, and ethyl palmitate] and aromatic carboxylic acid esters [aromatic monocarboxylic acid esters (monoesters), for example, esters of C₇₋₂₀ aromatic carboxylic acids, such as benzyl benzoate and benzyl salicylate].

A single kind of monocarboxylic acid ester or a combination of two or more kinds of monocarboxylic acid esters may be used.

### Ester of polyol having three or more hydroxy groups

The ester of a polyol having three or more hydroxy groups may be a partial ester (e.g., monoester or diester) or a complete ester (e.g., triester when the alcohol is a triol) and is typically a complete ester. The ester of a polyol having three or more hydroxy groups usually may be one that does not fall under the category of the "fat or oil" (such as MCTs).

In the case where the ester of a polyol having three or more hydroxy groups is an ester of two or more carboxylic acid molecules, the two or more carboxylic acid molecules may be the same or different molecules. The two or more carboxylic acid molecules are typically the same molecules.

The polyol having three or more hydroxy groups is not particularly limited, and examples include aliphatic polyols (including aromatic aliphatic polyols) and aromatic polyols. The polyol (aliphatic polyol) having three or more hydroxy groups may be saturated or unsaturated. The polyol (aliphatic polyol) having three or more hydroxy groups may be chained (including branched-chained) or cyclic.

The number of hydroxy groups in the polyol having three or more hydroxy groups is 3 or more and is, for example, 3 to 10, preferably 3 to 6, and more preferably 3 to 5 (e.g., 3).

Specific examples of the polyol having three or more hydroxy groups include aliphatic polyols {e.g., alkane tri- to hexa-ols [e.g., alkanetriols (e.g., C₃₋₁₀ alkanetriols such as glycerol, 1,2,4-butanetriol, trimethylolpropane, and trimethylolpropane), alkanetetraols (e.g., C₃₋₁₀ alkanetetraols such as erythritol and pentaerythritol), etc.], polyalkane polyols [e.g., poly(alkane tri- or hexa-ols) such as diglycerol, ditrimethylolpropane, and dipentaerythritol], etc.}.

The carboxylic acid is not particularly limited, and examples include aliphatic carboxylic acids (including aromatic aliphatic carboxylic acids) and aromatic carboxylic acids. The carboxylic acid may be saturated or unsaturated. The carboxylic acid may be chained (including branched-chained) or cyclic. The carboxylic acid may be, for example, an oxycarboxylic acid.

The carboxylic acid may be a monocarboxylic acid or a polycarboxylic acid and is typically a monocarboxylic acid.

Specific examples of the carboxylic acid (monocarboxylic acid) include aliphatic carboxylic acids (e.g., C₁₋₃₀ aliphatic carboxylic acids, preferably C₁₋₁₂ aliphatic carboxylic acids, more preferably C₁₋₆ aliphatic carboxylic acids, such as formic acid, acetic acid, propionic acid, butyric acid, valeric acid, isovaleric acid, caproic acid, enanthic acid, caprylic acid, pelargonic acid, capric acid, lauric acid, myristic acid, palmitic acid, margaric acid, stearic acid, oleic acid, and cyclohexane carboxylic acid) and aromatic carboxylic acids (e.g., carboxy C₆₋₁₀ arenes such as benzoic acid and salicylic acid).

Examples of the ester of a polyol having three or more hydroxy groups include esters of all combinations of these carboxylic acids and polyols.

Specific examples of the ester of a polyol having three or more hydroxy groups include triol esters {e.g., triol triesters [e.g., triesters of glycerol and aliphatic carboxylic acids (fatty acids) (e.g., triesters of glycerol and fatty acids having six or less carbon atoms (e.g., five or less carbon atoms, four or less carbon atoms, etc.), such as tri-C₁₋₆ fatty acid esters, tri-C₁₋₅ fatty acid esters, and tri-C₁₋₄ fatty acid esters), such as tributyrin], etc.}.

A single kind of ester of a polyol having three or more hydroxy groups or a combination of two or more kinds of esters of a polyol having three or more hydroxy groups may be used.

### Ester of polycarboxylic acid having three or more carboxyl groups

The ester of a polycarboxylic acid having three or more carboxyl groups may be a partial ester (e.g., monoester or diester) or a complete ester (e.g., triester when the polycarboxylic acid is a tricarboxylic acid) and is typically a complete ester.

In the case where the ester of a polycarboxylic acid having three or more carboxyl groups is an ester of two or more alcohol molecules, the two or more alcohol molecules may be the same or different molecules. The two or more alcohol molecules are typically the same molecules.

The polycarboxylic acid having three or more carboxyl groups is not particularly limited, and examples include aliphatic polycarboxylic acids (including aromatic aliphatic polycarboxylic acids). The polycarboxylic acid having three or more carboxyl groups may be saturated, unsaturated, or aromatic. The polycarboxylic acid having three or more carboxyl groups may be chained (including branched-chained) or cyclic.

The number of carboxyl groups in the polycarboxylic acid having three or more carboxyl groups is 3 or more and is, for example, 3 to 10, preferably 3 to 6, and more preferably 3 to 5 (e.g., 3).

Specific examples of the polycarboxylic acid having three or more carboxyl groups include aliphatic polycarboxylic acids {e.g., aliphatic tri- to hexa-carboxylic acids [e.g., poly (aliphatic tri- to hexa-carboxylic acids) such as aliphatic tricarboxylic acids (e.g., C₅₋₁₂ aliphatic tricarboxylic acids such as citric acid and aconitic acid)], etc.}.

The alcohol is not particularly limited, and examples include aliphatic alcohols (including aromatic aliphatic alcohols) and aromatic alcohols. The alcohol may be saturated or unsaturated. The alcohol may be chained (including branched-chained) or cyclic. The alcohol may be a monool or a polyol and is usually a monool.

Specific examples of the alcohol (monool) include aliphatic alcohols [e.g., alkanols (e.g., C₁₋₂₀ alkanols, preferably C₁₋₁₂ alkanols, more preferably C₁₋₆ alkanols (e.g., C₁₋₄ alkanols), such as methanol, ethanol, propanol, isopropanol, butanol, isobutanol, tert-butanol, pentanol, hexanol, heptanol, octanol, 2-ethyl hexanol, decanol, dodecanol, and cyclohexanol), aralkyl alcohols (e.g., hydroxy C₁₋₄ alkyl C₆₋₁₀ arenes such as benzyl alcohol and phenethyl alcohol), etc.].

Examples of the ester of a polycarboxylic acid having three or more carboxyl groups include esters of all combinations of these polycarboxylic acids and alcohols.

Specific examples of the ester of a polycarboxylic acid having three or more carboxyl groups include tricarboxylic acid esters {e.g., triesters of citric acid [e.g., triesters of citric acid and aliphatic alcohols (e.g., tri-C₁₋₆ alkyl citrates such as triethyl citrate), etc.], etc.}.

### Polyol ether

The polyol ether may be a partial ether (e.g., monoether) or a complete ether (e.g., diether when the polyalcohol is a diol) and is typically a complete ether.

In the case where the polyol ether is an ether of two or more alcohol molecules, the two or more alcohol molecules may be the same or different molecules. The two or more alcohol molecules are typically the same molecules.

The polyol is not particularly limited, and examples include aliphatic polyols (including aromatic aliphatic polyols) and aromatic polyols. The aliphatic polyol may be saturated or unsaturated. The aliphatic polyol may be chained (including branched-chained) or cyclic.

The number of hydroxy groups in the polyol is 2 or more and is, for example, 2 to 10, preferably 2 to 6, and more preferably 2 to 4 (e.g., 2).

Specific examples of the polyol include diols and polyols having three or more hydroxy groups.

Examples of the diol include aliphatic diols [e.g., alkanediols (e.g., C₂₋₂₀ alkanediols, preferably C₂₋₁₆ alkanediols, more preferably C₂₋₁₂ alcohols, such as ethylene glycol, 1,3-propanediol, propylene glycol, 1,4-butanediol, butylene glycol, pentanediol, hexanediol, heptanediol, octanediol, decanediol, dodecanediol, cyclohexanediol, and cyclohexanedimethanol), polyalkanediols (e.g., di- to hexa-C₂₋₆ alkanediols such as diethylene glycol, dipropylene glycol, and triethylene glycol), dihydroxyalkylarenes (e.g., di (hydroxy C₁₋₄ alkyl) C₆₋₁₀ arenes such as xylylene glycol), etc.].

Examples of the polyol having three or more hydroxy groups include aliphatic polyols {e.g., alkane tri- to hexa-ols [e.g., alkanetriols (e.g., C₃₋₁₀ alkanetriols such as glycerol, 1,2,4-butanetriol, trimethylolpropane, and trimethylolpropane), alkanetetraols (e.g., C₃₋₁₀ alkanetetraols such as erythritol and pentaerythritol), etc.], polyalkane polyols [e.g., poly(alkane tri- or hexa-ols) such as diglycerol, ditrimethylolpropane, and dipentaerythritol], etc.}.

The alcohol forming the ether group is not particularly limited, and examples include aliphatic alcohols (including aromatic aliphatic alcohols) and aromatic alcohols. The alcohol may be saturated or unsaturated. The alcohol may be chained (including branched-chained) or cyclic. The alcohol may be a monool or a polyol and is usually a monool.

Specific examples of the alcohol (monool) include aliphatic alcohols [e.g., alkanols (e.g., C₁₋₂₀ alkanols, preferably C₁₋₁₂ alkanols, more preferably C₁₋₆ alkanols (e.g., C₁₋₄ alkanols), such as methanol, ethanol, propanol, isopropanol, butanol, isobutanol, tert-butanol, pentanol, hexanol, heptanol, octanol, 2-ethyl hexanol, decanol, dodecanol, and cyclohexanol), aralkyl alcohols (e.g., hydroxy C₁₋₄ alkyl C₆₋₁₀ arenes such as benzyl alcohol and phenethyl alcohol), etc.].

Examples of the polyol ether include ethers of all combinations of these diols, polyols, and alcohols.

Specific examples of the polyol ether include aliphatic polyol ethers {e.g., aliphatic diol ethers [e.g., ethers (e.g., mono- or di-ethers with aliphatic alcohols, such as alkyl ethers (e.g., C₁₋₁₀ alkyl ethers, C₁₋₆ alkyl ethers, etc.)) of C₂₋₂₀ aliphatic diols, such as 1,2-diethoxyethane, ethylene glycol dibutyl ether, diethylene glycol diethyl ether, and diethylene glycol dibutyl ether], etc.}.

A single kind of polyol ether or a combination of two or more kinds of polyol ethers may be used.

### Polyamine

The number of amino groups (nitrogen atoms) in the polyamine is 2 or more and is, for example, 2 to 10, preferably 2 to 6, and more preferably 2 to 4 (e.g., 2).

The polyamine is not particularly limited and may be an aliphatic polyamine (including an aromatic aliphatic polyamine) or an aromatic polyamine. The aliphatic polyamine may be saturated or unsaturated. The aliphatic polyamine may be chained (including branched-chained) or cyclic.

The amino groups may be substituted amino groups. Examples of the substituent in the substituted amino group include hydrocarbon groups [e.g., alkyl groups (e.g., C₁₋₁₀ alkyl groups such as a methyl group, an ethyl group, a propyl group, an isopropyl group, a butyl group, an isobutyl group, a tert-butyl group, a hexyl group, and a 2-ethylhexyl group), etc.].

In the substituted amino group, one or two hydrogen atoms of the two hydrogen atoms forming the amino group are substituted.

Specific examples of the polyamine include polyamines (N-unsubstituted polyamines) [e.g., diamines (e.g., C₁₋₁₀ alkanediamines such as ethylenediamine, putrescine, cadaverine, and 1,6-hexanediamine)] and N-substituted polyamines [e.g., N-substituted diamines (e.g., N-mono- to tetra-alkyl (e.g., C₁₋₄ alkyl) C₁₋₁₀ alkanediamines such as N,N'-diethyl-1,6-hexanediamine)].

A single kind of polyamine or a combination of two or more kinds of polyamines may be used.

### Alcohol having six or more carbon atoms

The alcohol having six or more carbon atoms is not particularly limited, and examples include aliphatic alcohols (including aromatic aliphatic alcohols) and aromatic alcohols. Typically, the alcohol is an aliphatic alcohol.

The aliphatic alcohol may be saturated or unsaturated. The aliphatic alcohol may be chained (including branched-chained) or cyclic and is typically chained.

The alcohol having six or more carbon atoms may be a monool or a polyol and is usually a monool.

The alcohol having six or more carbon atoms may be a primary, secondary, or tertiary alcohol and is typically a primary alcohol.

Specific examples of the alcohol (monool) having six or more carbon atoms include aliphatic alcohols [e.g., alkanols (e.g., C₆₋₃₀ alkanols, preferably C₈₋₁₈ alkanols, more preferably C₁₀₋₁₆ alkanols, such as hexanol, heptanol, octanol, 2-ethyl hexanol, nonanol, decanol, undecanol, dodecanol, tridecanol, tetradecanol, pentadecanol, and hexadecanol), aralkyl alcohols (e.g., hydroxy C₁₋₄ alkyl C₆₋₁₀ arenes such as benzyl alcohol and phenethyl alcohol), etc.].

Typical examples of the alcohol having six or more carbon atoms include C₁₀₋₁₆ alcohols. C₁₀₋₁₆ alcohols are alcohols (e.g., straight-chained primary alcohols) having 10 to 16 carbon atoms.

Examples of the alcohol having six or more carbon atoms include C₁₀₋₁₆ alcohols [e.g., alkanols (in particular, straight-chained primary alcohols) such as 1-decanol, 1-undecanol, 1-dodecanol, 1-tridecanol, 1-tetradecanol, 1-pentadecanol, and 1-hexadecanol] and aralkyl alcohols (e.g., benzyl alcohol). 1-Decanol, benzyl alcohol, etc., are highly capable of dissolving menthol, but may have a slightly unusual odor as compared with other alcohols having six or more carbon atoms, although it depends on their amount contained in the composition. On the other hand, 1-tridecanol, 1-tetradecanol, 1-pentadecanol, and 1-hexadecanol have no odor, but are less capable of dissolving menthol than alcohols having a fewer carbon atoms, although it depends on their amount contained in the composition.

In the case of using alcohols having six or more carbon atoms, these issues may be taken into consideration to select an appropriate alcohol according to the desired application and other factors. For example, in the case of using benzyl alcohol, limiting its amount is desirable from the perspective of odor, and on top of that, combining with another ingredient (X) (e.g., a dicarboxylic acid diester) or another ingredient (e.g., an MCT) can achieve a balance between the dissolution of the menthol and the retention of menthol flavor. On the other hand, 1-dodecanol and others have no odor and are satisfactory in terms of the dissolution of the menthol, as compared to other alcohols having six or more carbon atoms, and are therefore particularly desirable.

A single kind of alcohol having six or more carbon atoms or a combination of two or more kinds of alcohols having six or more carbon atoms may be used.

In addition, the alcohol having six or more carbon atoms (e.g., C₁₀₋₁₆ alcohols) may be used in combination with an MCT and an ingredient (X) other than the alcohol having six or more carbon atoms. Such a combined use can provide a composition that advantageously achieves a balance between the dissolution of the menthol and the retention of menthol flavor.

In the case where the alcohol having six or more carbon atoms is used in combination with the ingredient (X) other than the alcohol having six or more carbon atoms (e.g., a dicarboxylic acid ester), the amounts of these ingredients can be selected according to the desired dissolution of the menthol, retention of menthol flavor, and other factors. For example, the amount of the alcohol having six or more carbon atoms relative to the combined amount of the alcohol having six or more carbon atoms and the ingredient (X) other than the alcohol having six or more carbon atoms may be 1 to 99 mass% (e.g., 2 to 98 mass%) and is preferably 5 to 95 mass% (e.g., 10 to 90 mass%). This amount may be 5 mass% or more, 10 mass% or more, 15 mass% or more, 20 mass% or more, 30 mass% or more, 40 mass% or more, 50 mass% or more, 95 mass% or less, 90 mass% or less, 85 mass% or less, 80 mass% or less, 75 mass% or less, 70 mass% or less, 60 mass% or less, 50 mass% or less, etc.

A single kind of ingredient (X) or a combination of two or more kinds of ingredients (X) may be used.

Among these ingredients (X), for example, dicarboxylic acid esters (e.g., dicarboxylic acid diesters such as aliphatic dicarboxylic acid diesters), diol esters (e.g., diol diesters such as diesters of aliphatic diols having three or more carbon atoms), monocarboxylic acid esters (e.g., esters of aliphatic monocarboxylic acids such as esters of C₆₋₂₄ aliphatic carboxylic acids), esters of polyols having three or more hydroxy groups (e.g., triol triesters such as aliphatic triol tri-C₁₋₆ fatty acid esters), polyol ethers [e.g., diol diethers such as aliphatic diol diethers (dialkyl ethers)], polyamines [e.g., diamines such as N-alkyl substituted diamines (e.g., N,N'-dialkyl diamines)], alcohols having six or more carbon atoms [e.g., C₁₀₋₁₆ alkanols (e.g., straight-chained alkanols such as 1-decanol, 1-dodecanol, and 1-hexadecanol), aralkyl alcohols (e.g., benzyl alcohol), etc.], etc., are suitable from the perspective of the dissolution of the menthol and others.

From the perspective of freezing resistance and others, dicarboxylic acid esters (e.g., dicarboxylic acid diesters such as aliphatic dicarboxylic acid diesters), monocarboxylic acid esters [e.g., aromatic monocarboxylic acid esters (monoesters), for example, esters of C₇₋₂₀ aromatic carboxylic acids, such as benzyl benzoate and benzyl salicylate], etc., are suitable.

Among these ingredients (X), dicarboxylic acid esters (e.g., aliphatic dicarboxylic acid diesters such as diesters of aliphatic dicarboxylic acids having four or more carbon atoms) are suitable from the perspective of compatibility (balance) between the dissolution and freezing resistance of the menthol and the retention of menthol flavor.

Therefore, the ingredient (X) may at least comprise an ingredient (compound) described above.

In the case where the ingredient (X) is used, the amount (concentration) of the ingredient (X) in the composition can be selected according to the mode of the composition, the type of ingredient (X), the application of the composition, and other factors, and is not particularly limited. The amount of the ingredient (X) in the composition can be selected from a range of about 99 mass% or less (e.g., 97 mass% or less) and is, for example, 95 mass% or less (e.g., 90 mass% or less), 85 mass% or less (e.g., 80 mass% or less), 75 mass% or less (e.g., 70 mass% or less), or 65 mass% or less (e.g., 60 mass% or less, 55 mass% or less, 50 mass% or less, 45 mass% or less, 40 mass% or less, 35 mass% or less, 30 mass% or less, 25 mass% or less, 20 mass% or less, etc.). In addition, the amount of the ingredient (X) in the composition is, for example, 1 mass% or more, 2 mass% or more, 3 mass% or more, 4 mass% or more, 5 mass% or more, 6 mass% or more, 7 mass% or more, 8 mass% or more, 9 mass% or more, 10 mass% or more, 12 mass% or more, 15 mass% or more, 18 mass% or more, 20 mass% or more, 22 mass% or more, 25 mass% or more, etc.

These upper limits and lower limits may be combined to set an appropriate range for the amount (concentration) of the ingredient (X) (e.g., 1 to 85 mass%, 5 to 50 mass%, 10 to 35 mass%, etc.), as described above.

The amount (concentration) of the ingredient (X) relative to the combined amount of the caryophyllene and the ingredient (X) [when the combined amount is assumed as 100 mass%] can be selected from the above-described concentration ranges of the ingredient (X) in the composition (e.g., 99 mass% or less, 90 mass% or less, 1 to 80 mass%, 3 to 70 mass%, 5 to 60 mass%, etc.).

Similarly, the amount (concentration) of the ingredient (X) relative to the combined amount of the menthol and the ingredient (X) [when the combined amount is assumed as 100 mass%] can be selected from the above-described concentration ranges of the ingredient (X) in the composition (e.g., 99 mass% or less, 90 mass% or less, 1 to 80 mass%, 3 to 70 mass%, 5 to 60 mass%, etc.).

Similarly, in the case where the composition comprises a fat or oil, the amount (concentration) of the ingredient (X) relative to the combined amount of the fat or oil and the ingredient (X) [when the combined amount is assumed as 100 mass%] can be selected from the above-described concentration ranges of the ingredient (X) in the composition (e.g., 99 mass% or less, 90 mass% or less, 1 to 80 mass%, 3 to 70 mass%, 5 to 60 mass%, etc.) again.

### Composition

In regard to the properties of the composition, the composition may be in a solid, liquid, or some other state (form), and is particularly in a liquid form. The liquid form includes a colloidal form, an emulsified form, and a gelatinous form.

The composition may be formulated into an appropriate dosage form according to the desired application, the mode of delivery, and other factors. The form (dosage form, properties) of the composition (formulation) is not particularly limited, and examples include tablets, powders, fine granules, granules, dry syrups, coated tablets, orally disintegrating tablets, chewable tablets, capsules, soft capsules, syrups, oral liquids, lozenges, jellies, inhalations, suppositories, injections, ointments, eye drops, eye ointments, nasal drops, ear drops, patches, lotions, topical liquids, sprays, topical aerosols, creams, gels, tapes, buccal tablets, sublingual tablets, liquids, suspensions, emulsions, liniments, and sheets.

The composition may be a pharmaceutical product (medicament, pharmaceutical composition).

In the case of delivery of the composition (or caryophyllene or menthol) into the body, the mode of delivery (administration or intake) is not particularly limited, and oral delivery (administration) or parenteral delivery (administration) may be selected. For parenteral delivery (administration), for example, pulmonary administration, nasal administration, dermal administration, mucosal (e.g., oral mucosal) administration, ocular instillation, auricular instillation, or injection (subcutaneous injection, intramuscular injection, intravenous injection, etc.) can be used. A single one of these modes of delivery or a combination of two or more of them may be used.

Typical modes of delivery include oral, pulmonary, or dermal administration. In particular, when the caryophyllene is intended to be delivered, preferred is pulmonary delivery. Pulmonary delivery (e.g., inhalation) enables efficient delivery of caryophyllene. For this reason, the mode of delivery may be at least pulmonary delivery.

The mode of delivery may be selected as appropriate according to the application and purpose of the composition to be delivered. For example, for purposes including achievement of at least one purpose (function, application) selected from (1) to (3) described later, pulmonary delivery is suitable because it seemingly allows the caryophyllene to efficiently (advantageously) achieve (exert) its function.

The amount of the composition delivered (administered, taken) can be selected according to the desired application/function and the dosage form (and the age, gender, and body weight of the subject, and other factors) and is not particularly limited.

For example, the composition may be delivered in a dose of 0.01 mg or more, 0.05 mg or more, 0.1 mg or more, etc., in terms of caryophyllene.

In a specific mode, the composition may be pulmonarily delivered (inhaled) at a rate of 0.1 mg/min or more in terms of caryophyllene.

The amount (volume) of the composition (caryophyllene-containing vapor or gas) per inhalation may be, for example, 10 mL or more, 20 mL or more, 30 mL or more, etc., and may be 4,500 mL or less, 4,000 mL or less, 3,000 mL or less, 2,000 mL or less, 1,000 mL or less, 500 mL or less, etc.

In another specific mode (e.g., for use in chewing tobacco), the composition may be orally delivered in a dose of, for example, 1 mg or more in terms of caryophyllene.

The frequency of delivery (e.g., the frequency of delivery per day) of the composition (caryophyllene) can be selected according to the mode of delivery, the desired function, and other factors, and may be once daily or several times daily.

The subject to which the composition is to be delivered may be, for example, a human or a non-human animal. The non-human animal may be a pet animal (e.g., a dog, a cat, etc.).

In the case where the composition comprises menthol, the mode of delivery of the menthol may be the same as that of the caryophyllene as described above.

The composition may be formulated into an appropriate dosage form as described above and can be used (applied) for various purposes (targets). Specific examples of the use (application) include capsules (e.g., capsule contents), filters, tobacco products, and inhalation devices.

These examples will be described below.

### Capsules

The capsule may have a shell only or have a shell and a content (core). In particular, the capsule for tobacco products, etc., may have a core (content, inner liquid, inner substance) and a shell (outer layer, coat, capsule coat).

The capsule may be a soft capsule, a hard capsule, a seamless capsule, etc. In particular, the capsule for tobacco products, etc., may be a seamless capsule.

The composition (caryophyllene, menthol, a fat or oil, an ingredient (X), and others) may be contained in a capsule in any manner, whether in a shell, a core, or both. In particular, when the capsule is a capsule having a core (seamless capsule), the composition may be contained in the core (or at least in the core). In other words, the composition of the present invention is used for a capsule content.

The shell (outer layer) usually may contain a shell-forming component (shell-forming base, shell-forming material). The shell-forming component is not particularly limited and can be selected as appropriate according to the application of the capsule and other factors. Examples of the shell-forming component include polysaccharides (or their derivatives) {e.g., polysaccharides from seaweeds [e.g., agar, carrageenan, alginic acid or its salts (e.g., alkali metal salts (a sodium salt, a potassium salt, etc.), alkaline earth metal salts (a calcium salt, a magnesium salt, etc.), an iron salt, a tin salt, and other metal salts), furcellaran, curdlan, etc.], polysaccharides from resins (e.g., gum ghatti, gum arabic, etc.), polysaccharides from microorganisms (e.g., pullulan, welan gum, xanthan gum, gellan gum, etc.), polysaccharides from plants (e.g., tragacanth gum, pectin, glucomannan, starch, polydextrose, dextrin, maltodextrin, cyclodextrin, indigestible dextrin, etc.), polysaccharides from seeds [e.g., guar gum or its derivatives (e.g., hydroxypropyl guar gum, cationic guar gum, and hydrolyzed guar gum (such as enzymatically hydrolyzed guar gum)), tara gum, tamarind seed gum, locust bean gum, psyllium seed gum, and linseed gum], fermented polysaccharides (e.g., diutan gum, etc.), cellulose derivatives (e.g., hydroxypropyl cellulose, hydroxypropylmethyl cellulose, methyl cellulose, carboxymethyl cellulose, etc.), chitosan, etc.}, synthetic resins (polyvinyl alcohol, etc.), proteins (e.g., gelatin, casein, zein, etc.), and sugar alcohols (e.g., sorbitol, maltitol, lactitol, hydrogenated isomaltulose, xylitol, mannitol, galactitol, erythritol, etc.).

A single kind of shell-forming component or a combination of two or more kinds of shell-forming components may be used.

The shell-forming component may be capable of forming hydrophilic colloids. Some types of shell-forming components can function, for example, as a plasticizer, a sweetener, a dietary fiber, or a bulking agent. The shell-forming component may be a commercial product.

The shell may contain a plasticizer, a colorant, a sweetener, a flavoring agent, an antioxidant, a preservative, and other components.

For example, the shell may contain a plasticizer for purposes including adjustment of shell strength. Specific examples of the plasticizer include polyhydric alcohols (e.g., (poly)alkylene glycols such as ethylene glycol, propylene glycol, polyethylene glycol, and polypropylene glycol; and polyols having three hydroxy groups or more, such as glycerol), sugars [e.g., monosaccharides (e.g., glucose, fructose, glucose, galactose, etc.), disaccharides (e.g., sucrose, maltose, trehalose, coupling sugar, etc.), oligosaccharides (e.g., maltooligosaccharides, etc.), etc.], sugar alcohols (e.g., sugar alcohols as exemplified above, such as sorbitol, maltitol, lactitol, hydrogenated isomaltulose, xylitol, mannitol, galactitol, and erythritol), polysaccharides or their derivatives [e.g., starch, starch derivatives (e.g., polydextrose, dextrin, maltodextrin, indigestible dextrin, cyclodextrins (α, β, and γ), etc.), cellulose derivatives (e.g., hydroxypropyl cellulose, hydroxypropylmethyl cellulose, methyl cellulose, carboxymethyl cellulose, etc.), etc.], polyvinyl alcohol, and triacetin. A single kind of plasticizer or a combination of two or more kinds of plasticizers may be used.

Sugar alcohols, starch, starch derivatives, etc., can be used also as the shell-forming component as described above.

In the capsule having a core, the core may be in a solid, liquid, or some other form. Particularly in the capsule containing the composition of the present invention as a core, the core may be in a liquid form. The liquid form includes a colloidal form, an emulsified form, and a gelatinous form.

The core contains at least caryophyllene (andmenthol and/or a fat or oil) as described above, and may contain an additional ingredient.

The additional ingredient is, for example, an ingredient (X) as exemplified above.

In such a capsule, the core is formed of the composition of the invention, and preferable modes, etc., of the core are as described above.

The core usually may be non-soluble in (non-erosive to) the shell (or to the area in contact with the shell).

The diameter (average diameter) of the capsule (or shell) can be selected as appropriate according to the type of capsule, the application, and other factors. The diameter may be, for example, 0.1 mm or more, 0.5 mm or more, 1 mm or more, 1.5 mm or more, 2 mm or more, etc. In addition, the diameter may be 30 mm or less, 25 mm or less, 20 mm or less, 18 mm or less, 15 mm or less, 12 mm or less, 10 mm or less, 8 mm or less, etc. More specifically, the diameter may be 2.8 mm, 3.0 mm, 3.4 mm, 3.5 mm, 4.0 mm, etc., but is not limited thereto.

The percentage of the shell in the capsule having a core (the proportion of the shell relative to the whole of the capsule (the combined amount of the shell and the capsule content)) may be selected from, for example, a range of about 0.1 to 99 mass% (e.g., 0.5 to 95 mass%). The shell percentage is, for example, about 1 to 90 mass%, preferably about 1.5 to 80 mass% (e.g., 2 to 70 mass%), and more preferably about 2.5 to 60 mass% (e.g., 3 to 50 mass%).

The thickness of the shell in the capsule having a core is not particularly limited and may be, for example, 1 to 200 µm, 3 to 150 µm, 5 to 100 µm, etc.

The capsule (e.g., the capsule having a core) may be breakable (disintegrable) (e.g., easily disintegrable or easily breakable). The crush strength of the capsule is determined according to the diameter and other factors, and may be, for example, 100 g or more, 200 g or more, 300 g or more, 400 g or more, 500 g or more, 600 g or more, 700 g or more, 800 g or more, 900 g or more, 1,000 g or more, etc.

The upper limit of the crush strength of the capsule is not particularly specified. The crush strength of the capsule may be, for example, 20,000 g or less, 15,000 g or less, 12,000 g or less, 10,000 g or less, etc.

The crush strength can be measured using, for example, a rheometer (CR-3000EX, manufactured by Sun Scientific).

The ratio of the crush strength (g) to the outer diameter (mm) (crush strength/outer diameter ratio) of the capsule (e.g., the capsule having a content) is not particularly limited and is, for example, 200 or more (e.g., more than 200), preferably 210 or more (e.g., 220 or more), more preferably 230 or more (e.g., 240 or more); or may be 250 or more, 300 or more, 400 or more, etc.

The upper limit of the ratio of the crush strength to the outer diameter (crush strength/outer diameter ratio) is not particularly specified and may be, for example, 20000, 15000, 10000, 8000, 6000, 5000, etc.

The ratio of the crush strength to the outer diameter can be said to be an index that reflects the practical breakability of the capsule, because there can be cases where the capsule is easily breakable even when the crush strength is high (for example, a case where the outer diameter is long).

The crush deformation of the capsule is determined according to the outer diameter and other factors, and may be, for example, 0.1 mm or more, 0.2 mm or more, 0.5 mm or more, 1.0 mm or more, etc.

The upper limit of the crush deformation of the soft capsule is not particularly specified. The crush deformation of the soft capsule may be, for example, 15 mm or less, 10 mm or less, 8 mm or less, etc.

The crush deformation can be measured using, for example, a rheometer (CR-3000EX, manufactured by Sun Scientific).

The ratio of the crush deformation (mm) to the outer diameter (mm) (crush deformation/outer diameter ratio) in the capsule is not particularly limited and is, for example, 0.1 or more, preferably 0.12 or more, more preferably 0.15 or more; or may be 0.18 or more, 0.2 or more, etc.

The upper limit of the ratio of the crush deformation to the outer diameter (crush deformation/outer diameter ratio) is not particularly specified and may be, for example, 1.0, 0.98, 0.97, 0.96, 0.95, etc.

The capsule may be used as it is or in combination with another capsule, depending on the application and other factors. The capsule may be, for example, embedded in a filter, as described later.

Said another capsule may be a capsule that does not contain caryophyllene. Said another capsule is, for example, a capsule having a core and a shell in which neither the core nor the shell contains caryophyllene.

Capsules (e.g., seamless capsules) can be produced using known methods. The capsule production methods are described in, for example, Japanese Patent No. 5047285, JP-A H10-506841, Japanese Patent No. 5581446, etc. More specifically, a drop-in-liquid method using a double or multiple nozzle can be employed. In this method, capsule shells are filled with a capsule content liquid, and the shells are then cured and dried to produce seamless capsules.

Such capsules can be used, for example, in filters, tobacco products, and inhalation devices, as described below, as well as in capsule formulations [e.g., capsules used for food and drink applications, such as health foods (e.g., food for specified health uses, food with nutrient function claims, etc.) and dietary supplements].

### Filters

The mode of use of the composition in filters is not particularly limited. For example, the composition may be contained in (attached to) various parts of a filter (filtration material, filtration member).

In particular, this type of filter may be a capsule-containing filter (a capsule-embedded filter, or a filter composed of a capsule-embedded filtration member).

In other words, this filter contains a capsule containing the composition (caryophyllene, menthol, a fat or oil, an ingredient (X), and others), which capsule is referred to as a first capsule. The first capsule can be, for example, the same capsule as described in the section "Capsules" above. The first capsule is particularly preferably a capsule having a core and a shell, which core (capsule content) contains (is formed of) the composition of the present invention.

The filter contains at least the first capsule, and may contain a second capsule, which is different from the first capsule.

The second capsule is not particularly limited as long as it is a capsule different from the first capsule. For example, the second capsule may be a capsule whose content is different from the content of the first capsule.

The second capsule is, for example, a capsule having a core and a shell, which core (and shell) contains a carrier and/or a flavoring agent (in particular, a capsule that does not contain caryophyllene).

The capsule contained in the above-described type of filter can be the same capsule as described in the section "Capsules" above. The capsule that does not contain caryophyllene (such as the second capsule) can be the same capsule as described in the section "Capsules" above except for the absence of caryophyllene.

The filter is not particularly limited and may be, for example, a filter for air conditioners, air purifiers, etc.

In particular, the capsule-containing filter is suitable for use as a tobacco filter, etc. In a mode of use in tobacco filters, etc., caryophyllene (and menthol) can efficiently be delivered via a pulmonary route.

The number of capsules in the filter, etc., can be selected as appropriate according to the application. The number of capsules in the filter, etc., may be one or two or more. As described above, the filter may contain the first capsule only (a single type of capsule), and may further contain the second capsule, which is different from the first capsule. The number of first capsules and the number of second capsules may be each one or two or more.

### Tobacco products

The mode of use of the composition in tobacco products is not particularly limited. For example, the composition may be contained in (attached to) various parts of a tobacco product (tobacco leaf-containing part, tobacco filter, etc.).

More specifically, the composition may be contained in tobacco leaf-containing parts, etc. {this type of mode is exemplified by, for example, cigarettes, cigars, pipes, *kiseru,* smokeless tobacco (e.g., chewing tobacco, snuff tobacco (snus, snuff, etc.), etc.}, or a capsule or filter containing the composition may be used in tobacco products.

Typically and preferably, a capsule or filter containing the composition is used in tobacco products.

As long as the capsule or filter is used in tobacco products, such tobacco products may be combustible tobacco products (e.g., cigarettes, cigars, pipes, *kiseru,* and bongs) or non-combustible tobacco products [e.g., heated tobacco products (direct heated, air heated, etc. smokeless tobacco, etc.].

### Inhalation devices

The mode of use of the composition in inhalation devices is not particularly limited. For example, the composition may be contained in (attached to) various parts of an inhalation device. The composition contained in the inhalation device may be in the form of a capsule containing the composition.

The inhalation device is not particularly limited, and examples include smoking devices.

Examples of the smoking device include heated tobacco products (such as vapor tobacco products), e-cigarettes, pipes, *kiseru,* bongs (water pipes, water cigarettes), and vaporizers. Heated tobacco products can be used for nicotine intake, while e-cigarettes are nicotine-free. The heated tobacco product is not particularly limited, and examples include IQOS (Philip Morris International), glo (British American Tobacco), Ploom S and Ploom TECH (Japan Tobacco), and Pulze (Imperial Tobacco Group plc) . The e-cigarette is not particularly limited, and examples include ego AIO (Joytech) and ICE VAPE (Common Wealth) .

More specifically, the composition (caryophyllene, menthol, and others), which is optionally encapsulated, may be contained in an inhalant in inhalation devices [e.g., a liquid solution in smoking devices such as heated tobacco products (such as vapor tobacco products), e-cigarettes, and bongs]. In a mode of use in inhalants, caryophyllene (and menthol) can efficiently be delivered via a pulmonary route.

The inhalant (such as a liquid solution) may contain an additional ingredient in addition to caryophyllene (and a fat or oil and/or menthol). The inhalant usually may contain a carrier [a liquid carrier, for example, a polyhydric alcohol (e.g., glycerol, propylene glycol, etc.)], and if necessary, may further contain a flavoring agent (may be a flavored liquid solution).

The amounts of the caryophyllene and other ingredients in the inhalant (such as a liquid solution) may be selected from the respective ranges as described above.

### EXAMPLES

Hereinafter, the present invention will be described in detail by examples, but the present invention is not limited thereto.

The ingredients used in the examples are as follows.
Caryophyllene (trade name: Caryophyllene AKY-2348, INABATA KORYO CO., LTD.)
MCT (trade name: Coconad ML, Kao Corporation)
Menthol (trade name: Menthol JP, Takasago International Corporation)

The physical properties of the capsules were measured or evaluated according to the following methods.

### Capsule crush strength and elasticity (crush deformation)

The crush strength of the capsules was measured using a rheometer CR-3000EX manufactured by Sun Scientific at room temperature (22 to 27°C) and 40 to 60% RH.

In the above measurement, the distance by which the capsule deformed until it crushed (the distance by which the capsule was compressed with the rheometer until the capsule crushed) was used as an index of the elasticity of the capsule.

### Capsule outer diameter

The outer diameter of the capsules was measured using a digital caliper manufactured by Mitutoyo Corporation (trade name: Quick Mini 25, model number: PK-0510SU, measurement range: 0 to 25 mm) at room temperature (22 to 27°C) and 40 to 60% RH.

### Capsule shell percentage

The percentage of the shell (shell percentage) was calculated as follows: shell percentage (%) = capsule shell mass / total capsule mass × 100.

The mass was measured using an electronic balance GX-200 manufactured by A&D Company, Limited.

### Capsule shell thickness

The thickness of the capsule shells (shell thickness) was measured using a digital microscope manufactured by Keyence Corporation (trade name: VHX-900, using a 10 µm calibration scale).

In the following examples, "%" refers to a percent by mass unless otherwise specified.

### Experiment 1: Volatilization of menthol

The compositions shown in the table below were prepared.

The prepared compositions were placed in petri dishes and used as test samples.

Each test sample was allowed to volatilize without being covered under room temperature conditions for a certain period of time. The mass of each test sample was measured at a given time point. The volatilization amount and rate were calculated from the measured values.

Similarly, the volatilization amount and rate of a test sample using only caryophyllene were also calculated.

The time constant was calculated from the measurement results [by subtracting the volatilization amount of caryophyllene (theoretical value)] .

The time constant can be defined, for example, as the time required for the mass (weight) of an ingredient to reach 1-e⁻¹ (or × 100%) of its initial concentration in an experimental system in which the ingredient decreases with time (e.g., the mass (weight) of a volatile ingredient decreases as a result of volatilization), when the remaining mass (weight) of the ingredient (the mass (weight) of the volatile ingredient remaining unvolatilized) is fitted as an exponential function of time by the least-squares method.

The results are shown in the table below. In the table, the unit of the time constant is hour (h) (the same applies hereinafter).

**[Table 1]**

| | | Experiment | | | | |
|---|---|---|---|---|---|---|
| | | 1A | 1B | 1C | 1D | 1E |
| Composition | Caryophyllene (%) | 0 | 15 | 34 | 59.5 | 85 |
| | Menthol (%) | 15 | 15 | 15 | 15 | 15 |
| | MCT (%) | 85 | 70 | 51 | 25.5 | 0 |
| Time constant | | 926.3 | 460.8 | 190.0 | 45.6 | 15.6 |

In the test samples, the amount of caryophyllene and the volatilization rate were positively correlated.

As is clear from the results in the above table, the same tendency was shown in the time constant calculated by subtraction of the volatilization amount of caryophyllene (theoretical value) calculated from the volatilization amount of the test sample using caryophyllene alone.

These results showed that caryophyllene enhances volatilization of menthol, which is a volatile ingredient (hardly volatile ingredient).

The enhancement of menthol volatilization will enhance the functionality and flavor of menthol.

Furthermore, caryophyllene itself volatilized, as is evident from the above experiment.

This means that when the above composition is, for example, volatilized for inhalation, not only menthol but also caryophyllene can efficiently be inhaled.

To confirm this, the following experiment was performed.

### Amount of volatilized caryophyllene during smoking cigarettes having caryophyllene-containing capsules embedded in cigarette filters

Easily disintegrable capsules having the composition of Experiment 1B as a content (core) were prepared by a drop method.

The capsules were designed to have a diameter of 3.4 mm, a shell thickness of 50 µm, a content mass of 19.3 mg, and a shell percentage of 13.4%.

The formula of the capsule shell was the same as that used in "Capsule Production Example 1" of Japanese Patent No. 6603817.

The capsules had a crush strength of 1,530 g and a crush deformation of 1.4 mm.

When each of the prepared capsules was picked and pressed with fingers, it broke easily with a snap, and the breaking sound and feel of the capsule were enjoyable. In addition, the flavor of menthol (and caryophyllene) was enjoyable.

Next, the capsules prepared above were placed in the center of cigarette filters.

In this experiment, the cigarette used was CORESTA Monitor 9 (CM9) from Borgwaldt GMBH.

The smoking machine used was Linear Smoking Machine (LM2) manufactured by Borgwaldt GMBH. Smoking was performed according to the method specified in ISO 3308 (35 mL puff of 2 second duration taken per minute).

Vapor and particulate ingredients of three cigarettes were adsorbed on a glass filter of the smoking machine. The amount of volatilized caryophyllene per cigarette was measured by GC/MS based on the method specified in ISO 10315. The amount of volatilized caryophyllene per cigarette was 0.29 mg.

This experiment confirmed that when a single cigarette having the capsule embedded in a cigarette filter was lit and smoked, 0.29 mg of volatilized caryophyllene was inhaled.

### Experiment 2: Dissolution of menthol

### Experimental method

The amounts (parts by mass (pbm)) of the ingredients shown in the table below were placed into 20-mL lidded glass bottles.

The lidded glass bottles were placed into a water bath set at 25°C. After the temperature reached 25°C, the bottles were kept warm for 30 minutes to adjust the temperature. After 30 minutes, menthol was added to each solution in the corresponding amount shown in the table below.

The temperature was continuously kept at 25°C. With every 20 minutes, the lidded glass bottles were inverted for mixing and checked visually to see if the menthol had dissolved. The time to complete dissolution was recorded as the time required for the menthol to dissolve. Check was made every 20 minutes until 360 minutes had passed, and then 3 days later, visual check was made again. When the menthol had not dissolved after 3 days, the result was recorded as non-dissolved.

In addition, with every 20 minutes, 10 µL of each solution was sampled and individually placed into a vial containing 990 µL of a standard solution for GC analysis. Sampling was done every 20 minutes until 360 minutes had passed, and then 3 days later, sampling was done again.

GC analysis was performed at a given time point under the conditions shown in the table below. Based on the measurement results obtained by GC analysis, the amount of menthol dissolved was fitted as an exponential function of time by the least-squares method, and the dissolution rate time constant was calculated.

**[Table 2]**

| Internal standard | |
|---|---|
| Component | Amount (pbm) |
| 2-Propanol | 950 |
| Dodecane | 20 |
| Heptadecane | 20 |

**[Table 3]**

| Conditions of GC analysis | |
|---|---|
| Instrument | GC-2014s, GC-2030 (Shimadzu Corporation) |
| Column | DB-Heavy WAX (Agilent Technologies) |
| Column temp. | 60.0°C |
| Vaporization chamber temp. | 240°C |
| Detector temp. | 250°C |
| Carrier gas | Helium |
| Injection vol. | 1.0µl |

The results are shown in the tables below.

**[Table 4]**

| | | Experiment | | | | | | | | | |
|---|---|---|---|---|---|---|---|---|---|---|---|
| | | 2A | 2B | 2C | 2D | 2E | 2F | 2G | 2H | 2I | 2J |
| Composition | Caryophyllene (%) | 0 | 95 | 0 | 5 | 40 | 60 | 80 | 0 | 15 | 75 |
| | MCT (%) | 95 | 0 | 80 | 75 | 40 | 20 | 0 | 75 | 60 | 0 |
| | Menthol (%) | 5 | 5 | 20 | 20 | 20 | 20 | 20 | 25 | 25 | 25 |
| Time required for dissolution (min) | | 100 | 40 | 160 | 160 | 80 | 80 | 80 | 280 | 200 | 100 |
| Dissolution rate time constant | | 17.3 | 9.0 | 13.1 | 9.2 | 9.0 | 8.2 | 7.7 | 25.4 | 19.7 | 11.6 |

**[Table 5]**

| | | Experiment | | | | | | |
|---|---|---|---|---|---|---|---|---|
| | | 2K | 2L | 2M | 2N | 2O | 2P | 2Q |
| Composition | Caryophyllene (%) | 0 | 0 | 70 | 15 | 0 | 15 | 55 |
| | MCT (%) | 70 | 0 | 0 | 45 | 55 | 40 | 0 |
| | Benzyl benzoate (%) | 0 | 70 | 0 | 0 | 0 | 0 | 0 |
| | Menthol (%) | 30 | 30 | 30 | 40 | 45 | 45 | 45 |
| Time required for dissolution (min) | | 340 | 140 | 120 | 3 days | Non-dis solved | 3 days | 220 |
| Dissolution rate time constant | | 29.8 | 25.3 | 15.5 | 24.5 | - | 26.9 | 6.2 |

As shown in the tables above, caryophyllene enhanced dissolution of menthol. A smaller time constant of the dissolution rate indicates a greater dissolution rate of menthol.

Such enhancement of menthol dissolution would be advantageous in terms of workability.

In addition, if menthol can be dissolved at 25°C as described above, there is no need for heating (e.g., at 40°C or 60°C), which advantageously reduces the number of work steps. It is also advantageous in that volatilization of menthol due to heating and changes in flavor due to heating can efficiently be prevented.

In particular, even a very high concentration of menthol (e.g., 40% or more, 45%, etc.) was successfully dissolved at 25°C with the addition of caryophyllene.

### Experiment 3: Freezing resistance

### Experimental method

The amounts (parts by mass (pbm)) of the ingredients shown in the table below were mixed and placed into 20-mL lidded glass bottles to dissolve into solutions. The lidded glass bottles were placed into a water bath set at 25°C. After the temperature reached 25°C, the bottles were kept warm for 30 minutes to adjust the temperature.

Then, the glass bottles were placed in a freezer (at approximately -20°C). The freezer was opened every 30 minutes to visually check if each solution had solidified. The time point when the solution was partially solidified and the time point when the solution was completely solidified were recorded. Check was made every 30 minutes until 360 minutes had passed, and then 3 days later, visual check was made again. When the menthol had not solidified after 3 days, the result was recorded as non-solidified.

The results are shown in the tables below.

**[Table 6]**

| | | Experiment | | | | | | | | | |
|---|---|---|---|---|---|---|---|---|---|---|---|
| | | 3A | 3B | 3C | 3D | 3E | 3F | 3G | 3H | 3I | 3J |
| Composition | Caryophyllene (%) | 0 | 20 | 40 | 40 | 0 | 40 | 0 | 40 | 0 | 40 |
| | MCT (%) | 100 | 80 | 60 | 30 | 0 | 0 | 0 | 0 | 0 | 0 |
| | Diethyl succinate (%) | 0 | 0 | 0 | 30 | 100 | 60 | 0 | 0 | 0 | 0 |
| | Diisobutyl adipate (%) | 0 | 0 | 0 | 0 | 0 | 0 | 100 | 60 | 0 | 0 |
| | Benzyl benzoate (%) | 0 | 0 | 0 | 0 | 0 | 0 | 0 | 0 | 100 | 60 |
| | Menthol (%) | 0 | 0 | 0 | 0 | 0 | 0 | 0 | 0 | 0 | 0 |
| Time to partial solidification (min) | 60 | 90 | 270 | 3 days | - | - | - | - | - | - | |
| Time to complete solidification (min) | 90 | 180 | 3 days | - | - | - | - | - | - | - | |

**[Table 7]**

| | | Experiment | | | | | |
|---|---|---|---|---|---|---|---|
| | | 3K | 3L | 3M | 3N | 3O | 3P |
| Composition | Caryophyllene (%) | 0 | 0 | 0 | 0 | 80 | 40 |
| | MCT (%) | 80 | 0 | 0 | 0 | 0 | 10 |
| | Diethyl succinate (%) | 0 | 80 | 0 | 0 | 0 | 10 |
| | Diisobutyl adipate (%) | 0 | 0 | 80 | 0 | 0 | 10 |
| | Benzyl benzoate (%) | 0 | 0 | 0 | 80 | 0 | 10 |
| | Menthol (%) | 20 | 20 | 20 | 20 | 20 | 20 |
| Time to partial solidification (min) | | 90 | 120 | 180 | 60 | 330 | 300 |
| Time to complete solidification (min) | | 180 | 150 | 3 days | 90 | 3 days | 3 days |

As is clear from the tables above, caryophyllene improved freezing resistance of the MCT.

In addition, caryophyllene showed excellent freezing resistance as a medium in menthol-containing compositions.

Furthermore, diethyl succinate and others showed freezing resistance when used in combination with caryophyllene.

Such compositions are less likely to solidify in cold regions and can efficiently be used in a wide range of regions. For example, when the above compositions are used as capsule contents, which are usually designed particularly not to solidify, solidification within the capsules can efficiently be prevented even in cold regions.

Diethyl succinate and others, which fall under the category of the aforementioned ingredient (X), were shown to have advantageous effects on, for example, dissolution of menthol (and retention of menthol flavor), and thus are suitable to be combined with caryophyllene. Experiment results on the dissolution of menthol, etc., are shown below.

### Experiment 4: Menthol dissolution test

For the menthol (l-menthol) dissolution test, a total of 10 g of menthol (l-menthol) and the other ingredients (solvent) was prepared and heated to 50°C to dissolve into a solution. The solution was stored at 20°C and evaluated based on the time during which the solution was maintained in a liquid state without solidifying.

A sample that solidified before being cooled down to 20°C [e.g., during the process of cooling down to a temperature below the melting point of l-menthol (generally 42 to 45°C)] or immediately (e.g., within 30 minutes) after being stored at 20°C was rated D. A sample that was maintained as a solution for a while (e.g., more than 30 minutes) but solidified in less than 24 hours was rated C. A sample that solidified in 24 hours or more but less than 10 days was rated B. A sample that did not solidify in 10 days or more was rated A.

In this experiment, the menthol used was a recrystallized product from steam-distilled essential oil from *Mentha canadensis,* which product was purchased from Anhui Tonghui Perfume Co., Ltd. The MCT used was a pressed oil product from *Elaeis guineensis* fruits, which product was purchased from Kao Corporation.

The results are shown below. In the tables, "parts by weight (pbw) " and "parts by mass (pbm) " have the same meaning.

**[Table 8]**

| Dissolution of l-menthol in diethyl malonate used as solvent | | | | |
|---|---|---|---|---|
| l-Menthol (pbw) | 80 | 70 | 60 | 50 |
| Diethyl malonate (pbw) | 20 | 30 | 40 | 50 |
| Dissolution | B | B | B | B |

**[Table 9]**

| Dissolution of l-menthol in diethyl succinate used as a solvent | | | | |
|---|---|---|---|---|
| l-Menthol (pbw) | 80 | 70 | 60 | 50 |
| Diethyl succinate (pbw) | 20 | 30 | 40 | 50 |
| Dissolution | C | A | A | A |

**[Table 10]**

| Dissolution of l-menthol in diisobutyl adipate used as a solvent | | | | |
|---|---|---|---|---|
| l-Menthol (pbw) | 80 | 70 | 60 | 50 |
| Diisobutyl adipate (pbw) | 20 | 30 | 40 | 50 |
| Dissolution | C | A | A | A |

**[Table 11]**

| Dissolution of l-menthol in diethyl sebacate used as a solvent | | | | |
|---|---|---|---|---|
| l-Menthol (pbw) | 80 | 70 | 60 | 50 |
| Diethyl sebacate (pbw) | 20 | 30 | 40 | 50 |
| Dissolution | C | A | A | A |

**[Table 12]**

| Dissolution of l-menthol in diethyl fumarate used as a solvent | | | |
|---|---|---|---|
| l-Menthol (pbw) | 70 | 60 | 50 |
| Diethyl fumarate (pbw) | 30 | 40 | 50 |
| Dissolution | C | A | A |

**[Table 13]**

| Dissolution of l-menthol in tributyrin used as a solvent | | | |
|---|---|---|---|
| l-Menthol (pbw) | 70 | 60 | 50 |
| Tributyrin (pbw) | 30 | 40 | 50 |
| Dissolution | C | C | B |

**[Table 14]**

| Dissolution of l-menthol in triethyl citrate used as a solvent | | | |
|---|---|---|---|
| l-Menthol (pbw) | 70 | 60 | 50 |
| Triethyl citrate (pbw) | 30 | 40 | 50 |
| Dissolution | C | C | C |

**[Table 15]**

| Dissolution of l-menthol in benzyl benzoate used as a solvent | | | |
|---|---|---|---|
| l-Menthol (pbw) | 70 | 60 | 50 |
| Benzyl benzoate (pbw) | 30 | 40 | 50 |
| Dissolution | C | C | B |

**[Table 16]**

| Dissolution of l-menthol in ethyl decanoate used as a solvent | | | |
|---|---|---|---|
| l-Menthol (pbw) | 70 | 60 | 50 |
| Ethyl decanoate (pbw) | 30 | 40 | 50 |
| Dissolution | C | A | A |

**[Table 17]**

| Dissolution of l-menthol in ethyl laurate used as a solvent | | | |
|---|---|---|---|
| l-Menthol (pbw) | 70 | 60 | 50 |
| Ethyl laurate (pbw) | 30 | 40 | 50 |
| Dissolution | C | A | A |

**[Table 18]**

| Dissolution of l-menthol in ethyl palmitate used as a solvent | | | |
|---|---|---|---|
| l-Menthol (pbw) | 70 | 60 | 50 |
| Ethyl palmitate (pbw) | 30 | 40 | 50 |
| Dissolution | C | B | B |

**[Table 19]**

| Dissolution of l-menthol in ethylene glycol diacetate used as a solvent | | | |
|---|---|---|---|
| l-Menthol (pbw) | 70 | 60 | 50 |
| Ethylene glycol diacetate (pbw) | 30 | 40 | 50 |
| Dissolution | C | C | C |

**[Table 20]**

| Dissolution of l-menthol in 1,6-diacetoxvhexane used as a solvent | | | |
|---|---|---|---|
| l-Menthol (pbw) | 70 | 60 | 50 |
| 1,6-Diacetoxyhexane (pbw) | 30 | 40 | 50 |
| Dissolution | C | A | A |

**[Table 21]**

| Dissolution of l-menthol in 1,8-diacetoxyoctane used as a solvent | | | |
|---|---|---|---|
| l-Menthol (pbw) | 70 | 60 | 50 |
| 1,8-Diacetoxyoctane (pbw) | 30 | 40 | 50 |
| Dissolution | C | C | A |

**[Table 22]**

| Dissolution of l-menthol in diethylene glycol dibutyl ether used as a solvent | | | |
|---|---|---|---|
| l-Menthol (pbw) | 70 | 60 | 50 |
| Diethylene glycol dibutyl ether (pbw) | 30 | 40 | 50 |
| Dissolution | C | A | A |

**[Table 23]**

| Dissolution of l-menthol in ethylene glycol dibutyl ether used as a solvent | | | |
|---|---|---|---|
| l-Menthol (pbw) | 70 | 60 | 50 |
| Ethylene glycol dibutyl ether (pbw) | 30 | 40 | 50 |
| Dissolution | A | A | A |

**[Table 24]**

| Dissolution of l-menthol in N,N'-diethyl-1,6-hexanediamine used as a solvent | | | |
|---|---|---|---|
| l-Menthol (pbw) | 70 | 60 | 50 |
| N,N'-Diethyl-1,6-hexanediamine (pbw) | 30 | 40 | 50 |
| Dissolution | A | A | A |

**[Table 25]**

| Dissolution of l-menthol in 1,2-diethoxyethane used as a solvent | | | |
|---|---|---|---|
| l-Menthol (pbw) | 70 | 60 | 50 |
| 1,2-Diethoxyethane (pbw) | 30 | 40 | 50 |
| Dissolution | A | A | A |

**[Table 26]**

| Dissolution of l-menthol in a mixture of diethyl succinate and MCT used as a solvent | | | | | | | | | |
|---|---|---|---|---|---|---|---|---|---|
| l-Menthol (pbw) | 70 | 70 | 70 | 65 | 65 | 65 | 60 | 60 | 60 |
| Diethyl succinate (pbw) | 10 | 15 | 20 | 10 | 15 | 20 | 10 | 15 | 20 |
| MCT (pbw) | 20 | 15 | 10 | 25 | 20 | 15 | 30 | 25 | 20 |
| Dissolution | C | C | B | C | C | A | A | A | A |

**[Table 27]**

| Dissolution of l-menthol in a mixture of diethyl succinate, 1-decanol, and MCT used as a solvent | | | | |
|---|---|---|---|---|
| l-Menthol (pbw) | 75 | 75 | 70 | 65 |
| Diethyl succinate (pbw) | 10 | 15 | 15 | 15 |
| 1-Decanol (pbw) | 15 | 10 | 10 | 2 |
| MCT (pbw) | 0 | 0 | 5 | 23 |
| Dissolution | A | A | A | A |

**[Table 28]**

| Dissolution of l-menthol in a mixture of diethyl succinate, 1-dodecanol, and MCT used as a solvent | | | | | | |
|---|---|---|---|---|---|---|
| l-Menthol (pbw) | 80 | 80 | 75 | 75 | 70 | 65 |
| Diethyl succinate (pbw) | 10 | 15 | 10 | 15 | 15 | 15 |
| 1-Dodecanol (pbw) | 10 | 5 | 15 | 10 | 10 | 10 |
| MCT (pbw) | 0 | 0 | 0 | 0 | 5 | 10 |
| Dissolution | A | A | A | A | A | A |

**[Table 29]**

| Dissolution of l-menthol in a mixture of 1-dodecanol with diethyl succinate, diisobutyl adipate, or diethyl sebacate used as a solvent | | | | | | | | | |
|---|---|---|---|---|---|---|---|---|---|
| l-Menthol (pbw) | 70 | 65 | 60 | 70 | 65 | 60 | 70 | 65 | 60 |
| Diethyl succinate (pbw) | 26 | 31 | 36 | 0 | 0 | 0 | 0 | 0 | 0 |
| Diisobutyl adipate (pbw) | 0 | 0 | 0 | 26 | 31 | 36 | 0 | 0 | 0 |
| Diethyl sebacate (pbw) | 0 | 0 | 0 | 0 | 0 | 0 | 26 | 31 | 36 |
| 1-Dodecanol (pbw) | 4 | 4 | 4 | 4 | 4 | 4 | 4 | 4 | 4 |
| Dissolution | A | A | A | A | A | A | B | B | B |

**[Table 30]**

| Dissolution of l-menthol in a mixture of diethyl succinate, 1-hexadecanol, and MCT used as a solvent | | | | |
|---|---|---|---|---|
| l-Menthol (pbw) | 75 | 75 | 70 | 65 |
| Diethyl succinate (pbw) | 10 | 15 | 15 | 15 |
| 1-Hexadecanol (pbw) | 15 | 10 | 10 | 2 |
| MCT (pbw) | 0 | 0 | 5 | 23 |
| Dissolution | A | B | A | A |

**[Table 31]**

| Dissolution of l-menthol in a mixture of benzyl alcohol and MCT used as a solvent | | | | | |
|---|---|---|---|---|---|
| l-Menthol (pbw) | 80 | 70 | 70 | 65 | 60 |
| Benzyl alcohol (pbw) | 20 | 5 | 10 | 5 | 5 |
| MCT (pbw) | 0 | 25 | 20 | 30 | 35 |
| Dissolution | A | A | A | A | A |

### Experiment 5: Odor comparison test A

For the odor comparison test A, a total of 10 g of menthol and the other ingredients (solvent) were prepared as a sample, and the odor of the sample was compared with that of an MCT solution of menthol (a mixture of 45 wt% menthol and 55 wt% MCT) in sensory testing. Sensory testing was performed by a single person, who rated samples based on the following criteria: when a sample smells the same as the MCT solution of menthol, the sample is regarded as having no unusual odor; when a sample smells weakly of what is different from menthol, the sample is regarded as having a weak unusual odor; and when a sample smells strongly of what is distinctively different from menthol, the sample is regarded as having a strong unusual odor.

The results are shown below.

**[Table 32]**

| | | | | |
|---|---|---|---|---|
| l-Menthol (pbw) | 80 | 70 | 60 | 50 |
| Diethyl succinate (pbw) | 20 | 30 | 40 | 50 |
| Odor (unusual odor) | None | None | None | None |

**[Table 33]**

| | | | | |
|---|---|---|---|---|
| l-Menthol (pbw) | 80 | 70 | 60 | 50 |
| Diisobutyl adipate (pbw) | 20 | 30 | 40 | 50 |
| Odor (unusual odor) | None | None | None | None |

**[Table 34]**

| | | | | |
|---|---|---|---|---|
| l-Menthol (pbw) | 80 | 70 | 60 | 50 |
| Diethyl sebacate (pbw) | 20 | 30 | 40 | 50 |
| Odor (unusual odor) | None | None | None | None |

**[Table 35]**

| | | | | | | | | | | | | |
|---|---|---|---|---|---|---|---|---|---|---|---|---|
| l-Menthol (pbw) | 70 | 70 | 70 | 70 | 65 | 65 | 65 | 65 | 60 | 60 | 60 | 60 |
| Diethyl succinate (pbw) | 10 | 15 | 20 | 25 | 10 | 15 | 20 | 25 | 10 | 15 | 20 | 25 |
| MCT (pbw) | 20 | 15 | 10 | 5 | 25 | 20 | 15 | 10 | 30 | 25 | 20 | 15 |
| Odor (unusual odor) | None | None | None | None | None | None | None | None | None | None | None | None |

**[Table 36]**

| | | | | | | |
|---|---|---|---|---|---|---|
| l-Menthol (pbw) | 80 | 80 | 75 | 75 | 70 | 65 |
| Diethyl succinate (pbw) | 10 | 15 | 10 | 15 | 15 | 15 |
| 1-Dodecanol (pbw) | 10 | 5 | 15 | 10 | 10 | 10 |
| MCT (pbw) | 0 | 0 | 0 | 0 | 5 | 10 |
| Odor (unusual odor) | None | None | None | None | None | None |

**[Table 37]**

| | | | | | | | | | |
|---|---|---|---|---|---|---|---|---|---|
| l-Menthol (pbw) | 70 | 65 | 60 | 70 | 65 | 60 | 70 | 65 | 60 |
| Diethyl succinate (pbw) | 26 | 31 | 36 | 0 | 0 | 0 | 0 | 0 | 0 |
| Diisobutyl adipate (pbw) | 0 | 0 | 0 | 26 | 31 | 36 | 0 | 0 | 0 |
| Diethyl sebacate (pbw) | 0 | 0 | 0 | 0 | 0 | 0 | 26 | 31 | 36 |
| 1-Dodecanol (pbw) | 4 | 4 | 4 | 4 | 4 | 4 | 4 | 4 | 4 |
| Odor (unusual odor) | None | None | None | None | None | None | None | None | None |

**[Table 38]**

| | | | | |
|---|---|---|---|---|
| l-Menthol (pbw) | 75 | 75 | 70 | 65 |
| Diethyl succinate (pbw) | 10 | 15 | 15 | 15 |
| 1-Hexadecanol (pbw) | 15 | 10 | 10 | 2 |
| MCT (pbw) | 0 | 0 | 5 | 23 |
| Odor (unusual odor) | None | None | None | None |

**[Table 39]**

| | | | | | | | | |
|---|---|---|---|---|---|---|---|---|
| l-Menthol (pbw) | 70 | 65 | 60 | 55 | 50 | 45 | 40 | 35 |
| MCT (pbw) | 30 | 35 | 40 | 45 | 50 | 55 | 60 | 65 |
| Odor (unusual odor) | None | None | None | None | None | None | None | None |

**[Table 40]**

| | | | | | |
|---|---|---|---|---|---|
| l-Menthol (pbw) | 80 | 70 | 70 | 65 | 60 |
| d-Limonene (pbw) | 20 | 5 | 10 | 5 | 5 |
| MCT (pbw) | 0 | 25 | 20 | 30 | 35 |
| Odor (unusual odor) | Strong | Strong | Strong | Strong | Strong |

**[Table 41]**

| | | | | | |
|---|---|---|---|---|---|
| l-Menthol (pbw) | 80 | 70 | 70 | 65 | 60 |
| Benzyl alcohol (pbw) | 20 | 5 | 10 | 5 | 5 |
| MCT (pbw) | 0 | 25 | 20 | 30 | 35 |
| Odor (unusual odor) | Strong | Weak | Weak | Weak | Weak |

### Experiment 6: Odor comparison test B

For the odor comparison test B, a total of 10 g of menthol and the other ingredients (solvent) were prepared as a sample, and the odor of the sample was compared with that of menthol in sensory testing.

Sensory testing was performed by eight well-trained persons, who rated samples based on the presence or absence of unusual odor on a scale up to 5 points. That is, when a sample smells the same as menthol (a mixture of 45 wt% menthol and 55 wt% MCT), the sample is given 5 points; when a sample smells weakly of what is different from menthol, the sample is given 3 points; when a sample smells strongly of what is distinctively different from menthol, the sample is given 1 point; when the average score is 4 points or more, the sample is rated A; when the average score is 3.5 points or more but less than 4 points, the sample is rated B; when the average score is 3 points or more but less than 3.5 points, the sample is rated C; when the average score is 2 points or more but less than 3 points, the sample is rated D; and when the average score is less than 2 points, the sample is rated E.

The results are shown below.

**[Table 42]**

| | | | | | | | |
|---|---|---|---|---|---|---|---|
| l-Menthol (pbw) | 70 | 60 | 50 | 50 | 50 | 60 | 70 |
| Diethyl malonate (pbw) | 30 | 40 | 50 | 0 | 0 | 0 | 0 |
| Diethyl succinate (pbw) | 0 | 0 | 0 | 50 | 0 | 0 | 0 |
| Diisobutyl adipate (pbw) | 0 | 0 | 0 | 0 | 50 | 0 | 0 |
| Diethyl sebacate (pbw) | 0 | 0 | 0 | 0 | 0 | 40 | 30 |
| Sensory test score | 3.00 | 3.00 | 2.25 | 4.00 | 3.50 | 4.25 | 4.13 |
| Rating | A | A | B | A | B | A | A |

**[Table 43]**

| | | | |
|---|---|---|---|
| l-Menthol (pbw) | 70 | 60 | 50 |
| Diethyl fumarate (pbw) | 30 | 40 | 50 |
| Sensory test score | 2.00 | 1.75 | 1.00 |
| Rating | D | E | E |

**[Table 44]**

| | | |
|---|---|---|
| l-Menthol (pbw) | 60 | 50 |
| Tributyrin (pbw) | 40 | 50 |
| Sensory test score | 3.88 | 3.50 |
| Rating | B | B |

**[Table 45]**

| | | | |
|---|---|---|---|
| l-Menthol (pbw) | 70 | 60 | 50 |
| Triethyl citrate (pbw) | 30 | 40 | 50 |
| Sensory test score | 4.00 | 3.75 | 3.63 |
| Rating | A | B | B |

**[Table 46]**

| | | | |
|---|---|---|---|
| l-Menthol (pbw) | 70 | 60 | 50 |
| Benzyl benzoate (pbw) | 30 | 40 | 50 |
| Sensory test score | 4.00 | 3.75 | 3.88 |
| Rating | A | B | B |

**[Table 47]**

| | | | |
|---|---|---|---|
| l-Menthol (pbw) | 70 | 60 | 50 |
| Diethylene glycol dibutyl ether (pbw) | 30 | 40 | 50 |
| Sensory test score | 2.25 | 2.50 | 2.25 |
| Rating | D | D | D |

**[Table 48]**

| | | |
|---|---|---|
| l-Menthol (pbw) | 70 | 50 |
| N,N'-Diethyl-1,6-hexanediamine (pbw) | 30 | 50 |
| Sensory test score | 1.63 | 1.25 |
| Rating | E | E |

**[Table 49]**

| | | | | |
|---|---|---|---|---|
| l-Menthol (pbw) | 80 | 80 | 80 | 80 |
| Diethyl succinate (pbw) | 10 | 15 | 0 | 0 |
| Diisobutyl adipate (pbw) | 0 | 0 | 10 | 15 |
| Dodecanol (pbw) | 10 | 5 | 10 | 5 |
| Sensory test score | 4.00 | 4.25 | 3.88 | 4.38 |
| Rating | A | A | B | A |

**[Table 50]**

| | | | |
|---|---|---|---|
| l-Menthol (pbw) | 60 | 50 | 40 |
| MCT (pbw) | 40 | 50 | 60 |
| Sensory test score | 4.13 | 4.75 | 4.63 |
| Rating | A | A | A |

**[Table 51]**

| | | | | | |
|---|---|---|---|---|---|
| l-Menthol (pbw) | 80 | 70 | 70 | 65 | 60 |
| d-Limonene (pbw) | 20 | 5 | 10 | 5 | 5 |
| MCT (pbw) | 0 | 25 | 20 | 30 | 35 |
| Sensory test score | 2.00 | 2.25 | 2.38 | 2.25 | 2.63 |
| Rating | D | D | D | D | D |

**[Table 52]**

| | | | | | |
|---|---|---|---|---|---|
| l-Menthol (pbw) | 80 | 70 | 70 | 65 | 60 |
| Benzyl alcohol (pbw) | 20 | 5 | 10 | 5 | 5 |
| MCT (pbw) | 0 | 25 | 20 | 30 | 35 |
| Sensory test score | 1.75 | 2.38 | 2.13 | 2.38 | 2.13 |
| Rating | E | D | D | D | D |

### Experiment 7: Encapsulation

As is clear from the results of Experiment 1 above, the composition used in the experiment was successfully encapsulated. Further experiments confirmed that all the compositions can form the same type of capsules as those in Experiment 1 with no problems.

Furthermore, additional experiments confirmed that, in addition to the type of capsule in Experiment 1 (capsule having a capsule diameter of 3.4 mm), the following four types of easily disintegratable capsules can be formed by a drop method for all the compositions. It should be noted that the formula of the capsule shell (outer layer) was the same among the capsules.
Capsule diameter: 2.8 mm, shell thickness: 57 µm, crush strength: 1,180 g, crush deformation: 1.5 mm
Capsule diameter: 3.0 mm, shell thickness: 48 µm, crush strength: 1,270 g, crush deformation: 1.6 mm
Capsule diameter: 3.5 mm, shell thickness: 48 µm, crush strength: 1,670 g, crush deformation: 1.8 mm
Capsule diameter: 4.0 mm, shell thickness: 45 µm, crush strength: 2,060 g, crush deformation: 2.0 mm

That is, all the compositions were successfully loaded into various seamless capsules (easily disintegratable capsules) by the drop method.

### INDUSTRIAL APPLICABILITY

The present invention provides a caryophyllene-containing composition and others.

## Claims

1. A composition comprising caryophyllene and menthol.

2. A composition comprising caryophyllene and a fat or oil.

3. A composition comprising caryophyllene, menthol, and a fat or oil.

4. The composition according to claim 2 or 3, wherein the fat or oil comprises a medium-chain triglyceride (MCT).

5. The composition according to any one of claims 1 to 4, wherein the amount of the caryophyllene is 1 mass% or more.

6. The composition according to any one of claims 1 and 3 to 5, wherein the amount of the caryophyllene is 3 mass% or more, and wherein the amount of the caryophyllene relative to the combined amount of the caryophyllene and the menthol is 5 to 95 mass%.

7. The composition according to any one of claims 2 to 6, wherein the amount of the caryophyllene is 3 mass% or more, and wherein the amount of the caryophyllene relative to the combined amount of the caryophyllene and the fat or oil is 5 to 95 mass%.

8. The composition according to any one of claims 3 to 7, wherein the amount of the caryophyllene is 3 mass% or more, and wherein the amount of the caryophyllene relative to the combined amount of the caryophyllene, the menthol, and the fat or oil is 5 to 95 mass%.

9. The composition according to any one of claims 1 and 3 to 8, wherein the amount of the menthol is 15 mass% or more.

10. The composition according to any one of claims 1 to 9, further comprising at least one ingredient (X) selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms.

11. The composition according to any one of claims 1 to 10, further comprising a dicarboxylic acid ester as the ingredient (X) .

12. The composition according to claim 10 or 11, wherein the amount of the ingredient (X) is 1 mass% or more.

13. The composition according to any one of claims 10 to 12, wherein the amount of the ingredient (X) is 3 mass% or more, and wherein the amount of the ingredient (X) relative to the combined amount of the caryophyllene and the ingredient (X) is 5 to 95 mass%.

14. The composition according to any one of claims 1 to 13, wherein the composition is in a liquid form.

15. The composition according to any one of claims 1 to 14, wherein the composition is intended for use for a capsule content.

16. A capsule having a core and a shell, wherein the core is formed of the composition according to any one of claims 1 to 15.

17. A filter at least containing a capsule, wherein the capsule has a core and a shell, and wherein the core is formed of the composition according to any one of claims 1 to 15.

18. A tobacco product containing the composition according to any one of claims 1 to 15.

19. An inhalation device containing the composition according to any one of claims 1 to 15.

20. The inhalation device according to claim 19, wherein the inhalation device is a smoking device.

21. The tobacco product or inhalation device according to any one of claims 18 to 20, wherein the tobacco product or inhalation device contains the capsule according to claim 16 or the filter according to claim 17.

22. An agent for enhancing volatilization of menthol, comprising caryophyllene.

23. An agent for enhancing dissolution of menthol, comprising caryophyllene.

24. An agent for enhancing freezing resistance of a fat or oil, comprising caryophyllene.

25. A method for enhancing volatilization and/or dissolution of menthol, comprising mixing caryophyllene with the menthol.

26. A method for enhancing freezing resistance of a fat or oil, comprising mixing caryophyllene with the fat or oil.

27. A method for pulmonary caryophyllene delivery, comprising using the capsule, the filter, the tobacco product, and/or the inhalation device according to any one of claims 16 to 21.
